# EUROPEAN PATENT APPLICATION

(11) **EP 2 889 382 A1**
(43) Date of publication of application: **01.07.2015**
(21) Application number: 13199813.0
(22) Date of filing: 30.12.2013
(51) Int. Cl.: C12Q 1/68

(54) **Oligonucleotides, use thereof, methods and a kit for detection of DNA Borrelia burgdorferi, Bartonella henselae, Babesia divergens, Anaplasma phagocytophilum**

(71) Applicant: Centrum Badan DNA Sp. z o.o., 61-612 Poznan (PL)
(72) Inventor: Wojciechowicz, Jacek, 61-612 Pozna (PL); Schmidt, Katarzyna, 61-612 Pozna (PL); Wróblewska-Kabba, Sylwia, 61-612 Poznan (PL); Boci g, Piotr, 61-612 Pozna (PL); Wojciechowicz, Tatiana, 61-612 Pozna (PL)
(74) Representative: Kossowska, Janina

(57) **Abstract**

The present invention relates to oligonucleotides, use thereof and methods and a kit for detecting the presence of DNAs of *Borrelia burdgoferi, Bartonella henselae, Babesia divergens, Anaplasma phagocytophilum* in the body fluids or tissue fragments by Real-Time PCR method.

SEQUENCE LISTING

<110> Centrum BadaÅ\204 DNA Sp z o.o.

<120> Oligonucleotides and use thereof,methods and kit for detecting
presence of DNA of Borrelia burgdorferi,
 Bartonella henselae, Babesia divergens, Anaplasma phagocytophilum

<160> 46

<170> BiSSAP 1.2

<210> 1
<211> 20
<212> DNA
<213> Artificial sequence

<220>
<221> source
<222> 1..20
<223> /organism="Artificial sequence"
      /note="oligonucleotide sequence of forward primer,
      complementary to the target B.burgdorferi sequence"
      /mol_type="unassigned DNA"

<400> 1
ccttcttttt gyacytcagc                                                  20


<210> 2
<211> 19
<212> DNA
<213> Artificial sequence
<220>
<221> source
<222> 1..19
<223> /organism="Artificial sequence"
      /note="oligonucleotide sequence of forward primer,
      complementary to the target B.burgdorferi sequence"
      /mol_type="unassigned DNA"

<400> 2
aagggcaagy aaagaggaa                                                   19


<210> 3
<211> 19
<212> DNA
<213> Artificial sequence

<220>
<221> source
<222> 1..19
<223> /organism="Artificial sequence"
      /note="AAGGGCAAGYAAAGAGGAA"
      /mol_type="unassigned DNA"

<400> 3
ttggaaaggr aagtcttat                                                   19


<210> 4
<211> 21
<212> DNA
<213> Artificial sequence

<220>
<221> source
<222> 1..21
<223> /organism="Artificial sequence"
      /note="oligonucleotide sequence of forward primer,
      complementary to the target B.burgdorferi sequence"
      /mol_type="unassigned DNA"

<400> 4
gatgggggaa tctcctgttg g                                                21


<210> 5
<211> 27
<212> DNA
<213> Artificial sequence

<220>
<221> source
<222> 1..27
<223> /organism="Artificial sequence"
      /note="oligonucleotide sequence of forward primer,
      complementary to the target B.burgdorferi sequence"
      /mol_type="unassigned DNA"

<400> 5
ccaaagttct gmaacattaa caccyaa                                          27


<210> 6
<211> 19
<212> DNA
<213> Artificial sequence

<220>
<221> source
<222> 1..19
<223> /organism="Artificial sequence"
      /note="oligonucleotide sequence of forward primer,
      complementary to the target B.burgdorferi sequence"
      /mol_type="unassigned DNA"

<400> 6
gaagtggtgg tgttgattt                                                   19


<210> 7
<211> 25
<212> DNA
<213> Artificial sequence

<220>
<221> source
<222> 1..25
<223> /organism="Artificial sequence"
      /note="oligonucleotide sequence of forward primer,
      complementary to the target B.burgdorferi sequence"
      /mol_type="unassigned DNA"

<400> 7
aacaagtaac yagatcrggc tcaag                                            25


<210> 8

<211> 21
<212> DNA
<213> Artificial sequence

<220>
<221> source
<222> 1..21
<223> /organism="Artificial sequence"
      /note="oligonucleotide sequence of forward primer,
      complementary to the target B.burgdorferi sequence"
      /mol_type="unassigned DNA"

<400> 8
tttggraarg gaagtcttat t                                                21


<210> 9
<211> 20
<212> DNA
<213> Artificial sequence

<220>
<221> source
<222> 1..20
<223> /organism="Artificial sequence"
      /note="oligonucleotide sequence of forward primer,
      complementary to the target B.burgdorferi sequence"
      /mol_type="unassigned DNA"

<400> 9
aggggtcgma taatagaaat                                                  20


<210> 10
<211> 18
<212> DNA
<213> Artificial sequence

<220>
<221> source
<222> 1..18
<223> /organism="Artificial sequence"
      /note="oligonucleotide sequence of forward primer,
      complementary to the target B.burgdorferi sequence"
      /mol_type="unassigned DNA"

<400> 10
cagaagtggt ggtgttga                                                    18


<210> 11
<211> 21
<212> DNA
<213> Artificial sequence

<220>
<221> source
<222> 1..21
<223> /organism="Artificial sequence"
      /note="oligonucleotide sequence of reverse primer,
      complementary to the target B.burgdorferi sequence"
      /mol_type="unassigned DNA"

<400> 11
tagatgargc tctyggcatt g                                                21

<210> 12
<211> 21
<212> DNA
<213> Artificial sequence

<220>
<221> source
<222> 1..21
<223> /organism="Artificial sequence"
      /note="oligonucleotide sequence of reverse primer,
      complementary to the target B.burgdorferi sequence"
      /mol_type="unassigned DNA"

<400> 12
agatkgggga atctyctgtt g                                                21


<210> 13
<211> 23
<212> DNA
<213> Artificial sequence

<220>
<221> source
<222> 1..23
<223> /organism="Artificial sequence"
      /note="oligonucleotide sequence of reverse primer,
      complementary to the target B.burgdorferi sequence"
      /mol_type="unassigned DNA"

<400> 13
gtcaaagtta aaggaaaaaa gag                                              23


<210> 14
<211> 22
<212> DNA
<213> Artificial sequence

<220>
<221> source
<222> 1..22
<223> /organism="Artificial sequence"
      /note="oligonucleotide sequence of reverse primer,
      complementary to the target B.burgdorferi sequence"
      /mol_type="unassigned DNA"

<400> 14
attagatgar gctctyggca tt                                               22


<210> 15
<211> 24
<212> DNA
<213> Artificial sequence

<220>
<221> source
<222> 1..24
<223> /organism="Artificial sequence"
      /note="oligonucleotide sequence of reverse primer,
      complementary to the target B.burgdorferi sequence"
      /mol_type="unassigned DNA"

<400> 15
aagtcttatt aagatkgggg aatc                                              24


<210> 16
<211> 20
<212> DNA
<213> Artificial sequence

<220>
<221> source
<222> 1..20
<223> /organism="Artificial sequence"
      /note="oligonucleotide sequence of reverse primer,
      complementary to the target B.burgdorferi sequence"
      /mol_type="unassigned DNA"

<400> 16
ttagatgarg ctctyggcat                                                   20


<210> 17
<211> 24
<212> DNA
<213> Artificial sequence

<220>
<221> source
<222> 1..24
<223> /organism="Artificial sequence"
      /note="oligonucleotide sequence of reverse primer,
      complementary to the target B.burgdorferi sequence"
      /mol_type="unassigned DNA"

<400> 17
aagcataatt taatacaaaa aaca                                              24


<210> 18
<211> 19
<212> DNA
<213> Artificial sequence

<220>
<221> source
<222> 1..19
<223> /organism="Artificial sequence"
      /note="oligonucleotide sequence of reverse primer,
      complementary to the target B.burgdorferi sequence"
      /mol_type="unassigned DNA"

<400> 18
cggtagtggt ctcgrgatt                                                    19


<210> 19
<211> 22
<212> DNA
<213> Artificial sequence

<220>
<221> source
<222> 1..22
<223> /organism="Artificial sequence"

/note="oligonucleotide sequence of reverse primer,
complementary to the target B.burgdorferi sequence"
/mol_type="unassigned DNA"

<400> 19
caayaccacc acttctratt aa                                                22

<210> 20
<211> 23
<212> DNA
<213> Artificial sequence

<220>
<221> source
<222> 1..23
<223> /organism="Artificial sequence"
/note="oligonucleotide sequence of reverse primer,
complementary to the target B.burgdorferi sequence"
/mol_type="unassigned DNA"

<400> 20
gccgctacag aatcaactac aat                                               23

<210> 21
<211> 21
<212> DNA
<213> Artificial sequence

<220>
<221> source
<222> 1..21
<223> /organism="Artificial sequence"
/note="oligonucleotide sequence of reverse primer,
complementary to the target B.burgdorferi sequence"
/mol_type="unassigned DNA"

<400> 21
ctcccatttc tccatctatc t                                                 21

<210> 22
<211> 18
<212> DNA
<213> Artificial sequence

<220>
<221> source
<222> 1..18
<223> /organism="Artificial sequence"
/note="oligonucleotide sequence of reverse primer,
complementary to the target B.burgdorferi sequence"
/mol_type="unassigned DNA"

<400> 22
ggtagtrgtc tcdggatt                                                     18

<210> 23
<211> 20
<212> DNA
<213> Artificial sequence

<220>

<221> source
<222> 1..20
<223> /organism="Artificial sequence"
      /note="oligonucleotide sequence of reverse primer,
      complementary to the target B.burgdorferi sequence"
      /mol_type="unassigned DNA"

<400> 23
tctcccaatg aataccawga                                                20


<210> 24
<211> 20
<212> DNA
<213> Artificial sequence

<220>
<221> source
<222> 1..20
<223> /organism="Artificial sequence"
      /note="oligonucleotide sequence of reverse primer,
      complementary to the target B.burgdorferi sequence"
      /mol_type="unassigned DNA"

<400> 24
gttacttgtt cgatctttcg                                                20


<210> 25
<211> 28
<212> DNA
<213> Artificial sequence

<220>
<221> source
<222> 1..28
<223> /organism="Artificial sequence"
      /note="oligonucleotide sequence of reverse primer,
      complementary to the target B.burgdorferi sequence"
      /mol_type="unassigned DNA"

<400> 25
cagaactttg gcttagtcag cctgatac                                       28


<210> 26
<211> 23
<212> DNA
<213> Artificial sequence

<220>
<221> source
<222> 1..23
<223> /organism="Artificial sequence"
      /note="oligonucleotide sequence of reverse primer,
      complementary to the target B.burgdorferi sequence"
      /mol_type="unassigned DNA"

<400> 26
ctccyaatga ataccaygar cct                                            23


<210> 27
<211> 28
<212> DNA

<213> Artificial sequence

<220>
<221> source
<222> 1..28
<223> /organism="Artificial sequence"
      /note="oligonucleotide sequence of the probe
        complementary to the target B.burgdorferi sequence"
      /mol_type="unassigned DNA"

<400> 27
ccygagtcgt ctggcaagac yactttaa                                          28


<210> 28
<211> 32
<212> DNA
<213> Artificial sequence

<220>
<221> source
<222> 1..32
<223> /organism="Artificial sequence"
      /note="oligonucleotide sequence of the probe
        complementary to the target B.burgdorferi sequence"
      /mol_type="unassigned DNA"

<400> 28
ttgtagttga ttcgtagcgg ctttaacccc ta                                     32


<210> 29
<211> 27
<212> DNA
<213> Artificial sequence

<220>
<221> source
<222> 1..27
<223> /organism="Artificial sequence"
      /note="oligonucleotide sequence of the probe
        complementary to the target B.burgdorferi sequence"
      /mol_type="unassigned DNA"

<400> 29
tccrgtatca ggctgactaa gccaaag                                           27


<210> 30
<211> 30
<212> DNA
<213> Artificial sequence

<220>
<221> source
<222> 1..30
<223> /organism="Artificial sequence"
      /note="oligonucleotide sequence of the probe
        complementary to the target B.burgdorferi sequence"
      /mol_type="unassigned DNA"

<400> 30
atcctgttta tgcaaaagct ttaggtgtta                                        30

<210> 31
<211> 30
<212> DNA
<213> Artificial sequence

<220>
<221> source
<222> 1..30
<223> /organism="Artificial sequence"
      /note="oligonucleotide sequence of the probe
        complementary to the target B.burgdorferi sequence"
      /mol_type="unassigned DNA"

<400> 31
aatacttgya ttatgtttat taatcaaata                                        30


<210> 32
<211> 27
<212> DNA
<213> Artificial sequence

<220>
<221> source
<222> 1..27
<223> /organism="Artificial sequence"
      /note="oligonucleotide sequence of the probe
        complementary to the target B.burgdorferi sequence"
      /mol_type="unassigned DNA"

<400> 32
tcgtctggca agacyacttt ractctt                                           27


<210> 33
<211> 28
<212> DNA
<213> Artificial sequence

<220>
<221> source
<222> 1..28
<223> /organism="Artificial sequence"
      /note="oligonucleotide sequence of the probe
        complementary to the target B.burgdorferi sequence"
      /mol_type="unassigned DNA"

<400> 33
atttctatta tkcgmcccct aggatatc                                          28


<210> 34
<211> 23
<212> DNA
<213> Artificial sequence

<220>
<221> source
<222> 1..23
<223> /organism="Artificial sequence"
      /note="oligonucleotide sequence of forward primer
        complementary to the target B.henselae sequence"
      /mol_type="unassigned DNA"

<400> 34

tttagaacgt tcgctacgat tgg                                              23


<210> 35
<211> 24
<212> DNA
<213> Artificial sequence

<220>
<221> source
<222> 1..24
<223> /organism="Artificial sequence"
      /note="oligonucleotide sequence of reverse primer
        complementary to the target B.henselae sequence"
      /mol_type="unassigned DNA"

<400> 35
aacggtcaaa gatgttccat taag                                             24


<210> 36
<211> 29
<212> DNA
<213> Artificial sequence

<220>
<221> source
<222> 1..29
<223> /organism="Artificial sequence"
      /note="oligonucleotide sequence of the probe
 complementary to the target B.henselae sequence"

      /mol_type="unassigned DNA"

<400> 36
tggtcatatt gatggtttgg ctgagatta                                        29


<210> 37
<211> 22
<212> DNA
<213> Artificial sequence

<220>
<221> source
<222> 1..22
<223> /organism="Artificial sequence"
      /note="oligonucleotide sequence of the forward primer
 complementary to the target B.divergens sequence"
      /mol_type="unassigned DNA"

<400> 37
ggaacatacc acggagacag tg                                               22


<210> 38
<211> 22
<212> DNA
<213> Artificial sequence

<220>
<221> source
<222> 1..22
<223> /organism="Artificial sequence"
      /note="oligonucleotide sequence of reverse primer,

complementary to the target B.divergens sequence"
      /mol_type="unassigned DNA"

<400> 38
tcctttagcc cagttgttac cg                                              22


<210> 39
<211> 30
<212> DNA
<213> Babesia divergens

<220>
<221> source
<222> 1..30
<223> /organism="Artificial sequence"
      /note="oligonucleotide sequence of the probe
      complementary to the target B.divergens sequence"
      /mol_type="unassigned DNA"

<400> 39
tatcggaaat ttatctcgcg cctgttaata                                      30


<210> 40
<211> 24
<212> DNA
<213> Artificial sequence

<220>
<221> source
<222> 1..24
<223> /organism="Artificial sequence
      /note="oligonucleotide sequence of forward primer,
      complementary to the target Anaplasma phagocytophilum sequence"
      /mol_type="unassigned DNA"

<400> 40
aaacattttc tggtgaacca atct                                            24


<210> 41
<211> 24
<212> DNA
<213> Artificial sequence

<220>
<221> source
<222> 1..24
<223> /organism="Artificial sequence
      /note="oligonucleotide sequence of reverse primer,
      complementary to the target A. phagocytophilum sequence"
      /mol_type="unassigned DNA"

<400> 41
aatttttata atgcacgcag atca                                            24


<210> 42
<211> 31
<212> DNA
<213> Artificial sequence

<220>
<221> source

<222> 1..31
<223> /organism="Artificial sequence
      /note="oligonucleotide sequence of the probe
      complementary to the target  A. phagocytophilum sequence"
      /mol_type="unassigned DNA"

<400> 42
aaataaacct gctccagaag atcctgtcat a                                    31


<210> 43
<211> 1097
<212> DNA
<213> Borrelia burgdorferi

<220>
<221> source
<222> 1..1097
<223> /organism="Borrelia burgdorferi"
      /mol_type="genomic DNA"

<400> 43
tgtcaaagtt aaaggaaaaa agagaaaaag ctgttgttgg catagaaagg gcaagtaaag      60

aggaagctat tgagcttgca agagttcaaa tagaaaaagc ttttggaaag ggaagtctta     120

ttaagatggg ggaatctcct gttggacaag gtataaaaag tatgtcaagt ggatctattg     180

tattagatga ggctctcggc attggcggat atcctagggg tcgcataata gaaatttttg     240

gccccgagtc gtctggcaag actactttaa ctcttcaagc gattgctgag gtgcaaaaag     300

aaggtgggat agctgctttt attgatgctg agcatgctct tgatcctgtt tatgcaaaag     360

ctttaggtgt taatgttgca gaactttggc ttagtcagcc tgataccgga gagcaagctc     420

ttgagattgc tgagcattta atcagaagtg gtggtgttga tttgattgta gttgattctg     480

tagcggcttt aacccctaaa ttagagatag atggagaaat gggagattct cagattggtc     540

ttcaagcaag gctaatgagc aaagctctta gaaagattac cggtatactt tctaaatcca     600

atacttgcat tatgtttatt aatcaaataa ggatgaggat tggtgttatg tttggtaatc     660

ccgagaccac taccggtggg aatgctttaa aattttattc atctcttaga cttgaggttc     720

gaaagatcga acaagtaact agatcgggct caagcgatga tgttattggc aataagatta     780

gagttaagat tgtaaagaac aaggttgctc caccttttcg taaagtagaa ttgataattt     840

attttgggaa aggtatttca agagaagctg gcattttaga tgctgctatt aagcataatt     900

taatacaaaa aacaggttca tggtattcat tgggagataa taagttggga caggggagag     960

agagtgttat tgagtatctt agcaaagaag tagaacttgc aaataatttg gataagaggc    1020

ttagaaaaat aatttttaat aattttgatc aagagaatga taattttatt gaatttaaag    1080

aagatgaatc tgagtaa                                                   1097


<210> 44
<211> 550
<212> DNA
<213> Bartonella henselae

<220>
<221> source
<222> 1..550
<223> /organism="Bartonella henselae"
      /mol_type="genomic DNA"

<400> 44
ggttgtgttg aagatattca atccttaaag caggggatgc gatttaatat ttctacacgt      60

tatagtatcg aaaacttaga aattggtgca tcaattgcgt gttcaggagt ttgtctaaca     120

attgttgagc gaggatcaaa acaacaagcg aagatcaatt ggtttgctgt agaagcgtgg     180

gaagaagcgt tgcgtttgac taatcttaca caatggacga aaggaacatt tattaattta     240

gaacgttcgc tacgattggg tgatgaaatg ggtgggcatt tggtttctgg tcatattgat     300

ggtttggctg agattattga tcagaaaagc gaaggtgatg cagttcgttt tttcctacaa     360

gtaccaaagc gttttatacc ttttattgta aataaaggtt ctatcgcact taatggaaca     420

tctttgaccg ttaattgtgt tgaagagcat attttcgatg ttcttattat tcgccataca     480

cttgaaatga caacctggag acaagctaaa attggagatc tggtcaattt agaaattgat     540

caacttgccc                                                            550


<210> 45
<211> 456
<212> DNA
<213> Babesia divergens

<220>
<221> source
<222> 1..456
<223> /organism="Babesia divergens"
      /mol_type="genomic DNA"

<400> 45
tgtggtaacc agatcggtgc caaattctgg gaggtcatct cagatgagca cggaatcgat      60

cccgtaagta ttatagcctt tctctgccat atgatagact gtgtgggcgt gtgctcacca     120

aaacacataa actacatgaa tgtgccgtgg tttgtgacaa atgccatgtc ttgcttcaca     180

atgtacttta ccaaagactt ttatattaaa tgcgctaaca acgtcacaga gcggaacata     240

ccacggagac agtgacctgc aactggagcg tgttgatgtg ttctacaatg aagccgctgg     300

tgggcgctac gtcccccgtg ccatcttgat ggacctggaa ccgggaacca tggactcagt     360

gcgcgcagga cctttcgggc agcttttcag gcctgataac ttcgtctttg gccagacggg     420

tgccggtaac aactgggcta aaggacacta cactga                               456


<210> 46
<211> 1243
<212> DNA
<213> Anaplasma phagocytophilum

<220>
<221> source

<222> 1..1243
<223> /organism="Anaplasma phagocytophilum"
      /mol_type="genomic DNA"

<400> 46
tattatatgg tagaaaaagc tgttttggag tgtggagacc gtaggattag tttgccggtt      60

ttacgcggca cggacgggac cccagtggtt gatatcacaa ccctatatag ggagaacaat     120

gtgctcacat atgacccggg ttttatgtcc actgctgctt gcagatcaga gataactttc     180

attgatggaa acaagggaac tctacgctac aggggtattg atatagaaaa tctgatcggt     240

acacccaata gcttcagtag tatagtgtat ttgttattga agggtactct gccttctgaa     300

actgagcatg aagagtttgt gcggattttg ggcgctgaat acgatgtgcc tgaacaagtt     360

atgaacgtta ttagatcatt ccctcgagat tcgcatccta tggctattct catagctagc     420

ttttctgctt tagctgctaa ttaccacgca agtcgcattg atccgcttac aggtgctatc     480

atcgcaattg cgaaagtacc cggcattgtt gcaagtattt ataggcacac tgcaaatcta     540

gattttatac aagctgacgc aaacttagag tacacgcatc actttatcag gatgatgttt     600

ggcgacatgg atgatgcaca tcgtgatatt atgcacaaag ctctagatgc aatttttata     660

atgcacgcag atcatgagca gaatgcctct acttccactg tgcgtatgac aggatcttct     720

ggagcaggtt tatttgcatg tttgtgcgca ggagtggcca ctctgtgggg gccagcgcat     780

gggggcgcaa atgaggctgt tataaagatg cttgctgaga ttggttcacc agaaaatgtt     840

tcgtcattta tcgacaaggt aaaaaacaac aagggcaaaa gtcgtcttat ggggtttggg     900

caccgtgttt ataaaagcta tgaccctaga gcgcgtgttt tgcgtacaat ctgtaaagat     960

gtattagatt ctctaggtag agatgagaat ttgctagctg ttgcagaaga gttagaaagt    1020

caggctttgc aggatgagta ttttattgag cgcaagttgt acccaaatgt cgacttttac    1080

tcaggtatag tgctaagagc tatgggggtt ccggtagata tgtatactgc attctttgcc    1140

ttggcacgga cgtctggatg ggcatctcaa tggtatgaga tgattcaaag tgatgaaggg    1200

aaacggatca gccgcccacg ccagttatac acaggaaaat aag                      1243

## Description

The invention relates to oligonucleotides, use thereof and methods and a kit for detecting the presence of DNA of *Borrelia burdgoferi, Bartonella henselae, Babesia divergens, Anaplasma phagocythophilum by* Real-Time PCR in body fluids or tissue fragments. Generally, the invention relates to amplification of nucleic acid. Oligonucleotides of the invention are used for DNA synthesis for detection and identification of genetic material of microorganisms in human body fluids or tissue fragments and are used in medical diagnostics.

### Borrelia burgdorferi

Lyme borreliosis (LB) is a tick-borne disease, transmitted by *Borrelia burgdorferi* sensu lato spirochetes (Burgdorfer, 1986, Rev. Infect. Dis. 6: 932-940). *B. burgdoferis strict, B. afzelii* and *B. garinii* are mainly responsible for dissemination of the disease in Europe (Steere, 2001, Lymedisease. N England J Med. 345:115-124).

According to Asbrink and Hovmark, LB is clinically divided into two phases, early and late. Early Lyme borreliosis is characterized by a limited infection (among others erythema migrans, lymphocytic lymphoma of the skin) and the stage of disseminated infection (among others multiple erythema migrans, early neuroborreliosis, arthritis, carditis). Late Lyme borreliosis is characterized by a stage of chronic changes (chronic atrophic dermatitis, neurologic, rheumatic changes, or in other organs for at least 12 months).

In Poland the presence of erythema migrans or the presence of antibodies against epitopes of *Borrelia burgdorferi* sensu lato was considered as a diagnostic criterion confirming borreliosis. A laboratory diagnosis of Lyme borreliosis up to the present has consisted in using one of three methods: i.e. ELISA, Western blotting or PCR. Direct detection of bacterial DNA by the PCR method is adopted for recognized a diagnostic method only when there are tested cerebrospinal fluid, synovial fluid or a skin biopsy from a place where there is erythema migrans, or the site of chronic atrophic dermatitis (ACA).

Detection of bacterial DNA in the blood beyond the early stage of the disease is considered to be unlikely, because of the belief that *Borrelia* avoid circulatory system. This view most likely results from low sensitivity applied research techniques (standard PCR). The present invention surprisingly shows that the reaction using fluorescent dye-labeled probes (sought microorganism gene fragment-specific) and using Real - Time PCR technique pathogen DNA can be detected in a biological sample from patients with chronic borreliosis.

The sequences of many specific regions of the genome of *Borrelia burgdorferi* have been known and they can be used for identification and differentiation thereof. Examples of such regions are genes: fla (encoding flagellin protein), recA, 16SrRNA, ospA, ospB.

Polymerase Chain Reaction (PCR) is an enzymatic method allowing for quick and selective multiplication of selected DNA sequence bordered by two oligonucleotide primers, complementary to opposite DNA strands (Saiki, et al Enzymatic amplification of beta-globin genomic sequences and restriction site analysis for diagnosis of sickle cell anemia. Science, Dec.20, 230 (4732): 1350-4, 1985, Saiki et al, Primer-directed enzymatic amplification of DNA with a thermostable DNA polymerase. Science. 239:487-491 1988, and European Patents EP 0200362 and EP0201184).

So far, there is no standardized procedure for detection of *Borrelia burgdorferi* infections by PCR. In addition, there is a lack of methods for identification of genetic material of bacteria in human peripheral blood. No method is available that allows for simultaneous determination of the level of *Borrelia burgdorferi* bacteremia.

There are several reports on the possibility of using standard PCR in *B.burgdorferi* diagnosis: WO9635450 (Diagnostic tests for a New spirochete, *Borrelia lonesterii*), U.S. Patent Application. 20070172829 wherein fragment of the 16S rRNA gene or gene encoding flagellin is used, and publications: Liebling, et al, "The Polymerase Chain Reaction for the Detection of Borrelia burgdorferi in Human Body Fluids," Arth Rheum 36 (5): 665-675, 1993, Schmidt et al," Detection of Borrelia burgdorferi DNA by Polymerase Chain Reaction in the Urine and Breast Milk of Patients with Lyme Borreliosis", Diagn. Microbiol. Infect. Dis. 21 :121-128, 1995; Burrascano, MD- Diagnostics Hints and Treatment Guidelines for Tick Borne Illnesses. - 12th edition, 1998; Schwaiger et al, "Routine diagnosis of Borrelia burgdorferi (sensu lato) infections using a real-time PCR assay " Clin. Microbiol. Infect. 7 (9): 461-9, 2001), U.S. Patent 5279938 (Sensitive diagnostic test for Lyme disease) refers to the use of PCR in the diagnosis of *B.burgdorferi,* although the sensitivity of the identification of the DNA was in the range of several hundred copies of the pathogen in the tested sample.

### Bartonella henselae

*Bartonella henselae* is a bacterium that lives mainly in erythrocytes. It is transferred to human body mainly by mammal blood-feeding arthropods, such as ticks, lice or fleas. An infection can also occur as a result of scratches or being bitten by an infected animal, usually a cat or a dog (hence also commonly called "cat scratch disease"). Initially, the disease was asymptomatic or with only local symptoms as redness or inflammation papule. Sometimes : fever, swollen lymph nodes, headaches, back pain, lower abdomen pain, and fatigue. If left untreated, the infection can lead to inflammation of brain or heart occur after a few weeks of infection.

### Bartonella henselae

The diagnosis of *Bartonella henselae* is based on a genetic method Real Time PCR a genetic method (identification and determination of the number of pathogen DNA copies), or t serological testing using the indirect immunofluorescence method (IFA) (the presence of specific antibodies of IgM and IgG classes).

### Anaplasma phagocytophihcm (which causes ehrlichiosis)

*Anaplasma phagocytophilum* is a Gram-negative bacterium that gets into the bloodstream along with the secretions of a tick. Infection can also occur perinatally or during exposure to infected blood. Researches have shown that in Europe in approximately 30% of patients infection coexists with tick borne encephalitis or Lyme borreliosis.

The most common symptoms include: a sudden onset of fever, severe headaches, chills, malaise, muscle pain. Less often there are anorexia, vomiting, nausea, abdominal pain, diarrhea, cough. In the case of acute infections, shock, similar to septic one, myocarditis, acute respiratory failure, renal failure, intravascular coagulation, and a number of neurological syndromes have been observed. The prognosis worsens with age, late application targeted antibiotic therapy and coexistence of other chronic diseases. The absence or occurrence of non-specific symptoms hinder correct diagnosis, and thus the full recuperation of the patient is not possible. The diagnosis of *Anaplasma phagocytophilum* is based on the genetic method Real Time PCR (identification and determination of the number of pathogen DNA copies) or serological testing with the method of indirect immunofluorescence (IFA) (the presence of specific antibodies of IgM and IgG classes).

### Babesia divergens (which causes babesiosis)

*Babesia divergens* belongs to a group of pathogenic protozoa transmitted by ticks and causing babesiosis (piroplasmosis). Babesiosis is a hemolytic disease similar to malaria, as the parasites attack the cells of erythrocytes. The disease usually manifests itself with fever, weakness and anorexia. Parasite infection can be severe, especially in immunocompromised individuals (children, the elderly people, or persons taking immunosuppressive drugs). Diagnosis of *Babesia divergens* is based on testing by genetic method Real Time PCR, which allows the detection of the presence of protozoa DNA.

The aim of the present invention is to provide a sensitive, highly specific and reliable method for detecting DNA of *Borrelia burdgoferi, Bartonella henselae, Babesia divergens, Anaplasma phagotyophilum* together or separately, and the means for its implementation which method would allow the development of the standard procedure for detection of infections with specific microorganisms on the basis of the blood sample drawn from patients or tissue sample from tick, allow for the simultaneous determination of the level of virulence of the detected strain. Surprisingly, the above objectives are achieved in the present invention.

The invention provides an oligonucleotide comprising at least 15 consecutive nucleotides belonging to an oligonucleotide derived from *Borrelia burgdorferi* recA sequence selected from oligonucleotides belonging to a group consisting of oligonucleotides RA_F1 to RA_F10 having the sequences presented in SEQ. ID. NO.: 1 to SEQ. ID. NO.: 10 or RA_R1 to RA_R16 having the sequences presented in SEQ. ID. NO.: 11 to SEQ. ID. NO.: 27 or RA_PR1 to RA_PR7 having the sequences shown in SEQ. ID. NO.: 28 to SEQ. ID. NO.: 33 and complementary sequences thereof. These oligonucleotides are also shown in Table 1, Table 2 and Table 3, in which their length and names as used hereinafter are given. Preferably, the oligonucleotide of the invention has the sequence of one of the recA oligonucleotides shown in Table 1 or Table 2 or Table 3, more preferably it is labeled, in particular it is fluorescently labeled.

**Table 1**

| **No.** | **The name of the oligonucleotide** | **Sequence of the oligonucleotide from the 5' to 3'** | **Oligonucleotide length [nt]** |
|---|---|---|---|
| 1 | RA_F1 | CCTTCTTTTTGC/(T)ACC/(T)TCAGC | 20 |
| 2 | RA_F2 | AAGGGCAAGT/(C)AAAGAGGAA | 19 |
| 3 | RA_F3 | TTGGAAAGGG/(A)AAGTCTTAT | 19 |
| 4 | RA_F4 | GATGGGGGAATCTCCTGTTGG | 21 |
| 5 | RA_F5 | CCAAAGTTCTGC/(A)AACATTAACACCT/(C)AA | 27 |
| 6 | RA_F6 | GAAGTGGTGGTGTTGATTT | 19 |
| 7 | RA_F7 | AACAAGTAACT/(C)AGATCG/(A)GGCTCAAG | 25 |
| 8 | RA_F8 | TTTGGA(/G)AAG/(A)GGAAGTCTTATT | 21 |
| 9 | RA_F9 | AGGGGTCGC/(A)ATAATAGAAAT | 20 |
| 10 | RA_F10 | CAGAAGTGGTGGTGTTGA | 18 |

The oligonucleotides shown in Table 1 may be used as forward primers in the methods of the invention.

**Table 2**

| **No.** | **The name of the oligonucleotide** | **Sequence of the oligonucleotide from the 5' to 3'** | **Oligonucleotide length [nt]** |
|---|---|---|---|
| 1 | RA_R1 | TAGATGAG(/A)GCTCTC(/T)GGCATTG | 21 |
| 2 | RA_R2 | AGATG/(T)GGGGAATCTC/(T)CTGTTG | 21 |
| 3 | RA_R3 | GTCAAAGTTAAAGGAAAAAAGAG | 23 |
| 4 | RA_R4 | ATTAGATGAG/(A)GCTCTC/(T)GGCATT | 22 |
| 5 | RA_R5 | AAGTCTTATTAAGATG/(T)GGGGAATC | 24 |
| 6 | RA_R6 | TTAGATGAG/(A)GCTCTC/(T)GGCAT | 20 |
| 7 | RA_R7 | AAGCATAATTTAATACAAAAAACA | 24 |
| 8 | RA_R8 | CGGTAGTGGTCTCGG/(A)GATT | 18 |
| 9 | RA_R9 | CAAC/(T)ACCACCACTTCTG/(A)ATTAA | 22 |
| 10 | RA_R10 | GCCGCTACAGAATCAACTACAAT | 23 |
| 11 | RA_R11 | CTCCCATTTCTCCATCTATCT | 21 |
| 12 | RA_R12 | GGTAGTG/(A)GTCTCG/(A,T)GGATT | 18 |
| 13 | RA_R13 | TCTCCCAATGAATACCAT/(A)GA | 20 |
| 14 | RA_R14 | GTTACTTGTTCGATCTTTCG | 20 |
| 15 | RA_R15 | CAGAACTTTGGCTTAGTCAGCCTGATAC | 28 |
| 16 | RA_R16 | CTCCC/(T)AATGAATACCAT/(C)GAA/(G)CCT | 23 |

The oligonucleotides shown in Table 2 may be used as reverse primers in the methods of the invention.

**Table 3**

| **No.** | **The name of the oligonucleotide** | **Sequence of the oligonucleotide from the 5' to 3'** | **Oligonucleotide length [nt]** |
|---|---|---|---|
| 1 | RA_PR1 | CCC/(T)GAGTCGTCTGGCAAGACT/(C)ACTTTAA | 28 |
| 2 | RA_PR2 | TTGTAGTTGATTCGTAGCGGCTTTAACCCCTA | 32 |
| 3 | RA_PR3 | TCCG/(A)GTATCAGGCTGACTAAGCCAAAG | 27 |
| 4 | RA_PR4 | ATCCTGTTTATGCAAAAGCTTTAGGTGTTA | 30 |
| 5 | RA_PR5 | AATACTTGC/(T)ATTATGTTTATTAATCAAATA | 30 |
| 6 | RA_PR6 | TCGTCTGGCAAGACT/(C)ACTTTA/(G)ACTCTT | 27 |
| 7 | RA_PR7 | ATTTCTATTATG/(T)CGC/(A)CCCCTAGGATATC | 28 |

The oligonucleotides shown in Table 3 may be used as the fluorescently labeled probes in the methods of the invention.

The invention also provides an oligonucleotide comprising at least 15 consecutive nucleotides, belonging to oligonucleotide derived from *Bartonella henselae* ribC sequence, selected from oligonucleotides belonging to a group consisting of oligonucleotides: BH_F1 having the sequence shown in SEQ. ID. NO.: 34, BH_R1 having the sequence shown in SEQ. ID. NO.: 35 and BH_PR1 having the sequence shown in SEQ. ID. NO.: 36 and the complementary sequences thereof.

These oligonucleotides are also shown in Table 4 or Table 5 or Table 6, in which their length and names as used hereinafter are given. Preferably, the oligonucleotide of the invention has the sequence of one of the oligonucleotides shown in Table 4 or Table 5 or Table 6 , optionally it is fluorescently labeled.

**Table 4**

| **No.** | **The name of the oligonucleotide** | **Sequence of the oligonucleotide from the 5' to 3'** | **Oligonucleotide length [nt]** |
|---|---|---|---|
| 1 | BH_F1 | TTTAGAACGTTCGCTACGATTGG | 23 |

The oligonucleotide shown in Table 4 can be used as a forward primer in the methods of the invention.

**Table 5**

| **No.** | **The name of the oligonucleotide** | **Sequence of the oligonucleotide from the 5' to 3'** | **Oligonucleotide length [nt]** |
|---|---|---|---|
| 1 | BH_R1 | AACGGTCAAAGATGTTCCATTAAG | 24 |

The oligonucleotide shown in Table 5 can be used as a reverse primer in the methods of the invention.

**Table 6**

| **No.** | **The name of the oligonucleotide** | **Sequence of the oligonucleotide from the 5' to 3'** | **Oligonucleotide length [\|nt]** |
|---|---|---|---|
| 1 | BH_Pr1 | TGGTCATATTGATGGTTTGGCTGAGATTA | 29 |

The oligonucleotide shown in Table 6 can be used as a fluorescently labeled probe in the methods according to the invention.

The invention also provides an oligonucleotide comprising at least 15 consecutive nucleotides, belonging to an oligonucleotide derived from the gene sequences for beta tubulin from *Babesia divergens,* selected from oligonucleotides comprising a group consisting of oligonucleotides: Bd_F1 having the sequence shown in SEQ. ID. NO.:37, Bd_R1 having the sequence shown in SEQ. ID. NO.:38 and Bd_Pr1 having the sequence shown in SEQ. ID. NO.:39 and complementary sequences thereof.

These oligonucleotides are also shown in Table 7 or Table 8 or Table 9, in which their length and the names used hereinafter are given. Preferably the oligonucleotide of the invention has the sequence of one of the oligonucleotides shown in Table 7 or Table 8 or Table 9, optionally it is fluorescently labeled.

**Table 7**

| **No.** | **The name of the oligonucleotide** | **Sequence of the oligonucleotide from the 5' to 3'** | **Oligonucleotide length [nt]** |
|---|---|---|---|
| 1 | Bd_F1 | GGAACATACCACGGAGACAGTG | 23 |

The oligonucleotide shown in Table 7 can be used as a forward primer in the methods of the invention.

**Table 8**

| **No.** | **The name of the oligonucleotide** | **Sequence of the oligonucleotide from the 5' to 3'** | **Oligonucleotide length [nt]** |
|---|---|---|---|
| 1 | Bd_R1 | TCCTTTAGCCCAGTTGTTACCG | 24 |

The oligonucleotide shown in Table 8 can be used as a reverse primer in the methods of the invention.

**Table 9**

| **No.** | **The name of the oligonucleotide** | **Sequence of the oligonucleotide from the 5' to 3'** | **Oligonucleotide length [nt] nt** |
|---|---|---|---|
| 1 | Bd_Pr1 | **TATCGGAAATTTATCTCGCGCCTGTTAA TA** | 30 |

The oligonucleotide shown in Table 9 can be used as a fluorescently-labeled probe in the methods of the invention.

The invention also relates to an oligonucleotide comprising at least 15 consecutive nucleotides, belonging to an oligonucleotide derived from the sequence of the gltA gene of *Anaplasma phagocytophilum,* which are selected from oligonucleotides belonging to a group consisting of oligonucleotides : Ap_F1 having the sequence shown in SEQ. ID. NO.:40, Ap_R1 having the sequence shown in SEQ. ID. NO.:41 and Ap_Pr1 having the sequence shown in SEQ. ID.. NO : 42 and the complementary sequences thereof. These oligonucleotides are also shown in Table 10 or Table 11 or Table 12 in which their length and the names used hereinafter are given. Preferably, the oligonucleotide according to the invention has the sequence of one of the oligonucleotides shown in Table 10 or Table 11 or Table 12, more preferably it is fluorescently labeled.

**Table 10**

| **No.** | **The name of the oligonucleotide** | **Sequence of the oligonucleotide from the 5' to 3'** | **Oligonucleotide length [nt]** |
|---|---|---|---|
| 1 | Ap_F1 | AAACATTTTCTGGTGAACCAATCT | 23 |

The oligonucleotide shown in Table 10 can be used as a forward primer in the methods of the invention.

**Table 11**

| **No.** | **The name of the oligonucleotide** | **Sequence of the oligonucleotide from the 5' to 3'** | **Oligonucleotide length [nt]** |
|---|---|---|---|
| 1 | Ap_R1 | AATTTTTATAATGCACGCAGATCA | 24 |

The oligonucleotide shown in Table 11 can be used as a reverse primer in the methods of the invention.

**Table 12**

| **No.** | **The name of the oligonucleotide** | **Sequence of the oligonucleotide from the 5' to 3'** | **Oligonucleotide length [nt]** |
|---|---|---|---|
| 1 | Ap_Pr1 | AAATAAACCTGCTCCAGAAGATCCTGTCATA | 31 |

The oligonucleotide shown in Table 12 can be used as a fluorescently labeled probe in the methods according to the invention.

Preferably, this oligonucleotide has a sequence of one of the oligonucleotides presented in SEQ. ID. NO: 1 - SEQ. ID. NO.:42, optionally it is fluorescently labeled.

The scope of the invention comprises also a method for detecting the presence and quantity of *Borrelia burgdorferi* DNA by Real-Time PCR in a tested biological sample comprising:
a) sample preparing;
b) isolation of the DNA;
c) amplification of a fragment of at least one sequence present in the *Borrelia burgdorferi* genome using the DNA contained in the test sample as a template, wherein the RecA gene-specific primers are oligonucleotide pair RA_F and RA_R selected from oligonucleotides comprising at least 15 contiguous nucleotides from RA_F1 to RA_F10 and RA_R1 to RA_R16 and having the sequences represented in SEQ. ID. NO: 1 to SEQ. ID. NO.:10 and SEQ. ID. NO.:11 to SEQ. ID. NO.:27;
d) identification of the increase of specific reaction products by detecting fluorescence level, derived from the probe selected from the oligonucleotides group RA_Pr1 to RA_Pr7 being fluorescently labeled having the sequences presented in SEQ. ID. NO.:28 to SEQ. ID. NO.:33, and
e) optionally determining the number of DNA copies of the test microorganism in a biological sample by comparing with a standard curve.

The invention also relates to a method for detecting the presence of *Borrelia burgdorferi* DNA by Real-Time PCR comprising:
a) sample preparing;
b) isolation of the DNA;
c) amplification of a fragment of at least one sequence present in the *Borrelia burgdoferi* genome using the DNA contained in the test sample as a template, wherein recA gene-specific primers are oligonucleotide pair RA_F and RA_R selected from oligonucleotides comprising at least 15 contiguous nucleotides from RA_F1 to RA_F10 and RA_R1 to RA_R16 and having the sequences shown in SEQ. ID. NO: 1 to SEQ. ID. NO: 10 and SEQ. ID. NO.:11 to SEQ. ID. NO.:27;
d) identification of the increase of specific reaction products by detecting fluorescence level derived from the intercalating fluorescent dye, located at the 5'-end and/or 3'-end or in the middle of the sequence of oligonucleotides used for the PCR reaction i.e. oligonucleotides having the sequences represented in SEQ. ID.NO: 1 to SEQ. ID. NO: 10 and SEQ. ID. NO.:11 to SEQ. ID. NO.:27, and
e) optionally determining the number of DNA copies of the test microorganism in a biological sample by comparing with a standard curve.

Furthermore, the invention relates to a method for detecting the presence and quantity of *B.henselae* DNA by Real- Time PCR in the test biological sample comprising:
a) sample preparing;
b) isolation of the DNA;
c) amplification of a fragment of at least one sequence present in the *B. henselae* genome, using DNA contained in the sample as a template, wherein the ribC gene-specific primers are oligonucleotide pair, each of them containing at least 15 contiguous nucleotides from the BH_F1 having the sequence shown in SEQ. ID. NO.:34 and BH_R1 having the sequence shown in SEQ. ID. NO.:35 ;
d) identification of an increase of the specific reaction products by detecting fluorescence level derived from the fluorescently labeled BH_Pr probe having the sequence shown in SEQ. ID. NO.:36, and
e) optionally determining the number of DNA copies of the test microorganism in a biological sample microorganism by comparing with a standard curve.

The invention further relates to a method for detecting the presence of the *B. henselae* DNA by the Real - Time PCR technique , which comprises:
a) sample preparing;
b) DNA isolation;
c) amplification of a fragment of at least one sequence present in the *B. henselae* genome using DNA contained in the test sample as a template, wherein the ribC gene-specific primers are oligonucleotide pair, each containing at least 15 contiguous nucleotides from the BH_F1 having the sequence shown in SEQ. ID. NO.:34 and BH_R1 having the sequence shown in SEQ. ID. NO.:35;
d) identification of the increase of specific reaction products by detecting fluorescence level derived from the intercalating fluorescent dye, located at the 5'-end and/or 3'-end or in the middle of the sequence of oligonucleotides used for the PCR reaction i.e. oligonucleotides having the sequences represented in SEQ. ID. NO.:34 and SEQ. ID. NO:35, and
e) optionally determining the number of DNA copies of the test microorganism in a biological sample by comparing with a standard curve.

In yet another embodiment the invention provides a method for detecting the presence and quantity of DNA *Babesia divergens* by Real -Time PCR technique in a test biological sample, which comprises:
a) sample preparing;
b) DNA isolation;
c) amplification of a fragment of at least one sequence present in the *B. divergens* genome using DNA contained in the test sample as a template, wherein the beta-tubulin gene-specific primers are a pair of oligonucleotides, each of them containing at least 15 contiguous nucleotides from the Bd_F1 having the sequence shown in SEQ. ID. NO.:37 and Bd_R1 having the sequence shown in SEQ. ID. NO.:38;
d) identification of an increase of specific reaction products by detecting fluorescence derived from the fluorescently labeled BH_Pr probe having the sequence shown in SEQ. ID. NO.:39, and
e) optionally determining the number of a DNA copies of the test microorganism in a biological sample by comparing with a standard curve.

The invention further provides a method for detecting the presence of *Babesia divergens* DNA by Real - Time PCR, which comprises:
a) sample preparing;
b) DNA isolation;
c) amplification of a fragment of at least one sequence present in the *Babesia divergens* genome using DNA contained in the sample as a template, wherein beta-tubulin gene-specific primers are a pair of oligonucleotides, each of them containing at least 15 contiguous nucleotides from the Bd_F1 having the sequence shown in SEQ. ID. NO.:37 and Bd_R1 having the sequence shown in SEQ. ID. NO.:38;
d) identification of an increase of the specific reaction products by detecting fluorescence level derived from the intercalating fluorescent dye, located at the 5'-end and/or 3'- end or in the middle of the sequence of oligonucleotides used for the PCR reaction i.e. oligonucleotides having the sequences represented in of SEQ. ID. NO.:37 and SEQ. ID. NO.:38, and
e) optionally determining the number of DNA copies of the test microorganism in a biological sample by comparing with a standard curve.

The scope of the invention also comprises a method for detecting the presence and amount of *Anaplasma phagocytophilum* DNA by Real-Time PCR technique in the test biological sample, comprising:
a) sample preparing;
b) DNA isolation;
c) amplification of a fragment of at least one sequence present in the *Anaplasma phagocytophilum* genome, using DNA contained in the test sample as a template, wherein gltA gene-specific primers are oligonucleotide pair, each of them containing at least 15 contiguous nucleotides from the Ap_F1 having the sequence shown in SEQ. ID. NO.:40 and the Ap_R1 having the sequence shown in the SEQ. ID. NO.:41;
d) identification of an increase in the specific reaction products by detecting fluorescence level derived from the fluorescently labeled probe Ap_Pr having the sequence shown in SEQ. ID. NO.:41, and
e) optionally determining the number of DNA copies of the test microorganism in a biological sample by comparing with a standard curve.

The invention further provides a method for detecting the presence of *Anaplasma phagocytophilum* DNA by Real-Time PCR technique, which comprises:
a) sample preparing;
b) DNA isolation;
c) amplification of a fragment of at least one sequence present in the *Anaplasma phagocytophilum* genome, using DNA contained in the sample as a template, wherein the gltA gene-specific primers are oligonucleotide pair, each of them containing at least 15 contiguous nucleotides from the Ap_F1 having the sequence shown in SEQ. ID. NO.:40, and Ap_R1 having the sequence presented in SEQ. ID. NO : 41;
d) identifying the increase of the specific reaction products by detecting fluorescence level derived from the fluorescent intercalating dye, located at the 5'-end and/or 3'-end, or in the middle of the sequence of oligonucleotides used for the PCR reaction i.e. oligonucleotides having the sequences set forth in SEQ. ID. NO.:40 and SEQ. ID. NO.:41, and
e) optionally determining the number of DNA copies of the test microorganism in the biological sample by comparing with a standard curve.

Another object of the invention is also a method for detecting the presence of DNA of the microorganisms from a group : *Borrelia burdgoferi*, *Bartonella henselae*, *Babesia divergens, Anaplasma phagocytophilum*

Real-Time PCR technique, comprising:
a) sample preparing;
b) DNA isolation;
c) amplification reaction of:
   - fragment of the recA gene sequence (SEQ. ID. NO.:43) present in the Borrelia burgdorferi genome of using the DNA contained in the test sample as a template, wherein the recA-specific primers are a pair of oligonucleotides RA_F and RA_R selected from oligonucleotides RA_F1 to RA_F10 and RA_R1 to RA_R16 having the sequences presented in Table 1 or Table 2, and a probe is preferably labeled with a fluorescent dye and is selected from the probes: RA_PR1 to RA_PR7 having the sequence represented in Table 3 and
   - fragment of the ribC gene sequence (SEQ. ID. NO.:44 ) present in the Bartonella henselae genome using DNA contained in the test sample as a template, wherein the ribC gene-specific primers are the oligonucleotide pair BH_F1 and BH_R1 having the sequences shown in Table 4 or Table 5 and a probe BH_Pr1 preferably labeled with a fluorescent dye, having the sequence shown in Table 6 and
      fragment of the beta tubulin gene sequence (SEQ. ID. NO.:5 ) present in the Babesia divergens genome using DNA in the test sample as a template, wherein the beta tubulin gene-specific primers are oligonucleotide pair Bd_F1 and Bd_R1 having sequences shown in Table 7 or Table 8 and Bd_Pr1 probe is used preferably labeled with a fluorescent dye, having the sequence shown in Table 9, and
      fragment of the gltA gene sequence (SEQ. ID. NO.:46) present in the Anaplasma phagocytophilum genome using DNA contained in a test sample as a template, wherein the gltA gene-specific primers are the oligonucleotide pair Ap_F1 and Ap_R1 having the sequences shown in Table 10 or Table 11, and Ap_Pr1 probe is used preferably labeled with a fluorescent dye, having the sequence shown in Table 12,
d) and then identifying increase the specific reaction products by detecting the fluorescence level derived from the fluorescently labeled probes;
e) optionally determining the number of DNA copies of the tested microorganisms in a biological sample by comparing with the a standard curve.

In a preferred embodiment of the method according to the invention as a primer pair with the corresponding fluorescently labeled probe, the primer pair shown in Table 13 is used. Preferably the set represented in Table 1 and Table 2 is used as primers, and preferably the labeled oligonucleotides, in particular fluorescently labeled, having the sequences shown in Table 3 are used as a probe.

Table 13. An exemplary set of primers, probes and fluorescent dyes used in the Real-Time PCR reaction for identification of B. burgdorferi DNA in the test samples

| **No.** | **Primer Name** | **Sequence of primers and probes from 5' to 3'** | **The dye/guencher** | **Product PCR length [bp]** |
|---|---|---|---|---|
| 1 | RA_F1 | CCTTCTTTTTGC/(T)ACC/(T)TCAGC | TET | 126 |
| | RA-R1 | TAGATGAG(/A)GCTCTC(/T)GGCATTG | FAM | |
| 2 | RA_F5 | CCAAAGTTCTGC/(A)AACATTAACACCT/(C)AA | --- | 277 |
| | RA_R5 | AAGTCTTATTAAGATG/(T)GGGGAATC | --- | |
| | RA_PR7 | ATTTCTATTATG/(T)CGC/(A)CCCCTAGGATATC | FAM/ TAMRA | |
| 3 | RA_F4 | GATGGGGGAATCTCCTGTTGG | --- | 553 |
| | RA_R8 | CGGTAGTGGTCTCGG/(A)GATT | **---** | |
| | RA_PR1 | CCC/(T)GAGTCGTCTGGCAAGACT/(C)ACTTTAA | **---** | |
| 4 | RA_F2 | AAGGGCAAGT/(C)AAAGAGGAA | TET/TAMRA | 412 |
| | RA_R9 | CAAC/(T)ACCACCACTTCTG/(A)ATTAA | -**--** | |
| 5 | RA_F2 | AAGGGCAAGT/(C)AAAGAGGAA | -**--** | 412 |
| | RA_R9 | CAAC/(T)ACCACCACTTCTG/(A)ATTAA | -**--** | |
| | RA_PR6 | TCGTCTGGCAAGACT/(C)ACTTTA/(G)ACTCTT | HEX | |
| 6 | RA_F10 | CAGAAGTGGTGGTGTTGA | --- | 236 |
| | RA_R8 | CGGTAGTGGTCTCGG/(A)GATT | -**--** | |
| | RA_PR2 | TTGTAGTTGATTCGTAGCGGCTTTAACCCCTA | TET | |
| 7 | RA_F4 | GATGGGGGAATCTCCTGTTGG | -**--** | 791 |
| | RA_R7 | AAGCATAATTTAATACAAAAAACA | -**--** | |
| | RA_PR1 | CCC/(T)GAGTCGTCTGGCAAGACT/(C)ACTTTAA | FAM/BHQ | |

In the preferred embodiment of the method according to the invention oligonucleotides having the sequences shown in Table 1 and/or Table 2 are used as a pair of primers, preferably labeled with a dye, particularly with a fluorescent dye. Preferably, in the methods of the present invention, one of DNA intercalating dyes (SybrGreen, Eva Greek, SYBR Gold, SYTO9) or another having the similar physicochemical properties is used as a fluorescent dye.

Preferably, in the methods of the invention initial denaturation at 95°C 1-5 min, then 25-55 thermal cycles with a profile: 95°C - 20 s , 58-63°C - 20 s and 72°C - 30 s are used for amplification , and readings of the fluorescence level are taken after each reaction cycle.

Preferably, the amplification products are detected in a real time by using probes and/or the fluorescently stained primers or fluorescent dyes intercalating amplified DNA and apparatus for Real-Time PCR. Preferably, the test sample is obtained from tested patient's blood, preferably whole blood , serum, plasma, or protein-cell pellet after centrifugation of plasma, buffy coat, or the cellular fraction of blood nucleated cells or cerebrospinal fluid or synovial fluid, or urine, or tissue biopsy.

Preferably, the test sample is obtained from a tick tissue or animal tissue.

Preferably, the test sample is obtained from the blood of a test subject during the course of antibiotic therapy in order to verify the effectiveness of therapy.

The methods of the invention as disclosed above are used to detect DNA of an individual microorganisms such as *Borrelia burgdoferi,* and to characterize the strain identified on the basis of Real-Time PCR, or for the simultaneous detection of DNA of *Borrelia burdgoferi, Bartonella henselae, Babesia divergens, Anaplasma phagotyhophilum.* Each of the methods consists of the same steps :
- an appropriate biological sample preparation;
- isolation of total DNA from the test sample;
- amplification by Real-Time PCR technique a sequence of tested microorganism/s using a set of primers or primers and probes allowing amplification DNA fragments specific for that bacteria;
- detection of amplicons in the test samples by measuring the fluorescence emission.

The above methods for the first time provide not only the possibility to detect microorganisms in the all stages of infection, but also to determine the precise amount thereof in different biological samples. Simultaneous detection of various microorganisms in one biological sample provides more comfort to the patient, and is also important from an economic point of view. In certain cases it can also be performed to detect a single microorganism. The invention also relates to a kit for detecting the presence of DNA of *B.burgdorferi* and/or *B.henselae* and/or *B.divergens,* and/or *A.phagocytophilum,* which comprises:
- at least two different oligonucleotides, each comprising at least 15 consecutive nucleotides comprised byo an oligonucleotide derived from the sequence of the *B.burgdorferi* recA genome, one selected from a group consisting of RA_F1 to RA_F10 having the sequences represented in SEQ ID N NO: 1 to SEQ. ID. NO.: 10 and the second one selected from a group consisting of RA_R1 to RA_R16 having the sequences shown in SEQ. ID. NO.:11 to SEQ. ID. NO.:27 and the labeled oligonucleotide selected from RA_PR1 to RA_PR7 having the sequences shown in SEQ. ID. NO.:28 to SEQ. ID. NO.:33, and the complementary sequences thereof,
- and/or
- at least two different oligonucleotides, each comprising at least 15 consecutive nucleotides comprised by the oligonucleotide derived from the sequence of the *B*. *henselae* ribC genome, selected from BH_F1 oligonucleotide having the sequence shown in SEQ. ID. NO.:34 and the oligonucleotide BH_R1 having the sequence shown in SEQ. ID. NO.:35 and the labeled BH_Pr having the sequence shown in SEQ. ID. NO.:36 and the complementary sequences thereof,
   and/or
- at least two different oligonucleotides, each comprising at least 15 consecutive nucleotides comprised by the oligonucleotide derived from the sequence of the beta tubulin gene from the *B.divergens* genome selected from Bd_F1 oligonucleotide having the sequence shown in SEQ. ID. NO.:37, and Bd_R1 oligonucleotide having the sequence shown in SEQ. ID. NO.:38 and the fluorescently labeled Bd_Pr having the sequence shown in SEQ. ID. NO.:39 and the complementary sequences thereof, and/or
- at least two different oligonucleotides, each comprising at least 15 consecutive nucleotides comprised by the oligonucleotide derived from the gltA gene sequence of *A*. *phagocytophilum* genome, selected from the Ap_F1 oligonucleotide having the sequence shown in SEQ. ID. NO.:40, and Ap_R1 oligonucleotide having the sequence shown in SEQ. ID. NO.:41 and the labeled Ap_Pr1 having the sequence shown in SEQ. ID. NO.:42 and the complementary sequences thereof
wherein the invention further comprises also dyes and optionally suitable reagents for DNA amplification. The kit can be used to detect a single pathogen, or all of the metioned pathogens simultaneously.

Preferably, the kit is for the detecting of DNAs of *B.burgdoiferi* and/or *B. henselae* and/or *B. divergens,* and/or *A*. *phagocytophilum* in biological samples of patients with late stage Lyme borreliosis.

Preferably, the kit is for the detecting of DNAs of *B.burgdorferi* and/or *B. henselae* and/or *B. divergens,* and/or *A*. *phagocytophilum* in biological samples of patients during the course of antibiotics therapy to verify the effectiveness of treatment.

The probe or the primers in the kit are fluorescently labeled.

The invention also relates to the use of the oligonucleotide comprising at least 15 consecutivenucleotides comprised by oligonucleotide derived from the RecA sequence of the *B.burgdorferi* genome selected from the group consisting of oligonucleotides : RA_F1 to RA_F10 having the sequences shown in SEQ. ID. NO: 1 to SEQ. ID. NO.: 10 or RA_R1 to RA_R16 having the sequences shown in SEQ. ID. NO.: 11 to SEQ. ID. NO.:27 or RA_PR1 to RA_PR7 having the sequences shown in SEQ. ID. NO.:28 to the SEQ. ID. NO.:33 and the complementary sequences thereof;
and/or
derived from the the ribC sequence of *B. henselae* genome selected from the group consisting of oligonucleotides BH_F1 having the sequence shown in SEQ. ID. NO.:34, BH_R1 having the sequence shown in the SEQ. ID. NO.:35 and BH_Pr having the sequence shown in SEQ. ID. NO.:36 and the complementary sequence thereof
and/or
derived from the beta- tubulin gene sequence from *B.divergens* genome selected from a group consisting of Bd_F1 oligonucleotides having the sequence shown in SEQ. ID. NO.:37, Bd_R1 having the sequence shown in SEQ. ID. NO.:38 and Bd_Pr having the sequence shown in SEQ. ID. NO.:39, and the complementary sequences thereof;
and/or
derived from the gltA gene sequence of the *A*. *phagocytophilum* genome, selected from a group consisting of Ap-F1 oligonucleotides having the sequence shown in SEQ. ID. NO.:40, Ap_R1 having the sequence shown in SEQ. ID. NO.:41 and Ap_Pr1 having the sequence shown in SEQ. ID. NO.:42 and the complementary sequences thereof,
for the preparation of a kit for detecting the presence of DNAs of *Borrelia burgdorferi* and/or *B.henselae* and/or *B.divergens* and/or *A.phagocytophilum* and diagnosis of infections with these microorganism.

Detection of microorganism infections at the DNA level allows for very early detection of pathogen presence , even before the appearance of specific antibodies against antigen of test - pathogen. The present invention has a number of further advantages that distinguish it from the previously known methods, which and available in the art. It enables diagnostics for microorganism/s with a high level of sensitivity, which is important for the course of treatment, because the method according to the invention identifies infection at a very early stage allowing to undertake therapy earlier. Nowadays routinely used serological tests detect the specific antibodies, which are produced by the body only after 4-6 weeks of infection. The invention also allows to diagnose patients in the late phase of Lyme borreliosis, where (in approximately 20-30 % of patients) the antibodies against this bacterium are not produced (seronegative), so that they can be diagnosed using serological tests (ELISA, Western blot).

Surprisingly, the invention allows for the quantitative determination of the level of *B. burgdorferi* bacteremia and to monitor the bacterial DNA level during antibiotic treatment, confirming the effectiveness of the therapy.

To better illustrate the essence of the invention, this description is enriched by a sequence listing and figures.

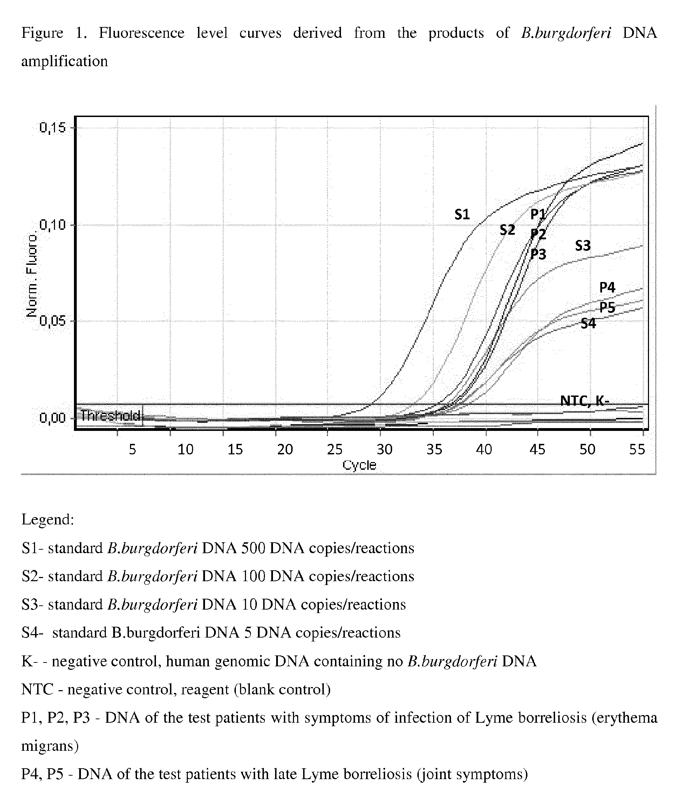

**Figure 1** shows the fluorescence level curves derived from *B.burgdorferi* DNA amplification products. Abbreviations: S1- standard *B.burgdorferi* DNA 500 DNA copies of /reactions; S2 -standard *B.burgdorferi* DNA 100 DNA copies/reactions ; S3 - standard 10 DNA copies/reactions ; S4 - standard 5 DNA copies/reactions, K- negative control, human genomic DNA containing no *B.burgdorferi* DNA; NTC - negative, reagent control, ( blank control); P1 , P2 , P3 - DNA of the patients with symptoms of infection of Lyme borreliosis (erythema migrans);, P4, P5 - DNA of the patients with late stage Lyme borreliosis (joint symptoms).

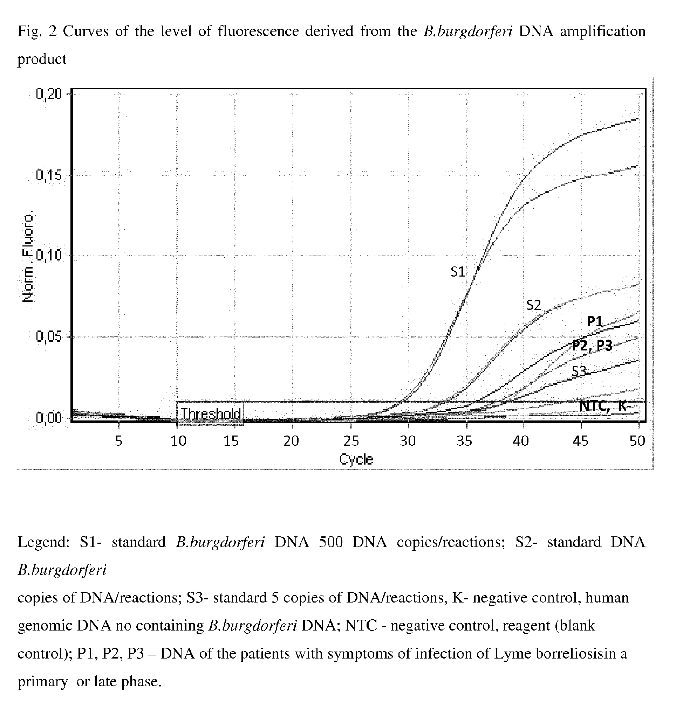

**Figure 2** shows curves of the fluorescence level derived from the products of *B.burgdoiferi* DNA amplification. Abbreviations: S1- standard DNA *B.burgdorferi* 500 DNA copies/reactions; S2 - standard *B.burgdorferi* DNA 50 DNA copies/reactions; S3 - standard 5 DNA copies/reactions; K- negative control, human genomic DNA containing no *B.burgdorferi* DNA, NTC - negative control, reagent (blank control);, P1, P2 , P3 - DNA of the patients with symptoms of infection of Lyme borreliosis in the initial or late stage.

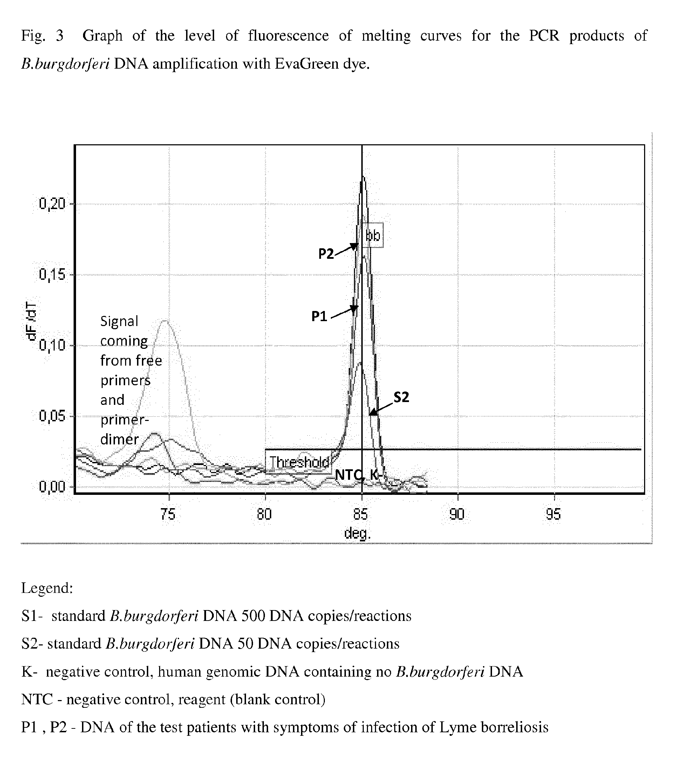

**Figure 3** shows the fluorescence level graph of melting curves for the products from *B. burgdorferi* DNA PCR amplification using dye EvaGreen.
   Abbreviations: S1- standard *B.burgdorferi* DNA 500 DNA copies/reactions; S2- standard *B.burgdorferi* DNA 50 DNA copies/reactions; K- negative control, human genomic DNA containing no *B.burgdorferi* DNA; NTC - negative control, reagent (blank control) , P1, P2 - DNA of patients with symptoms of Lyme borreliosis infection.

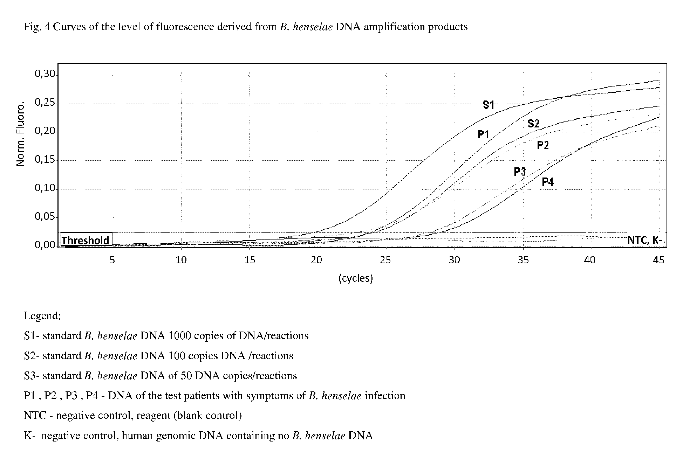

**Figure 4** shows the fluorescence level curves derived from the products of *Bartonella henselae* DNA amplification. Abbreviations: S1- standard *Bartonella henselae* DNA 100 DNA copies /reactions; K- negative control, human genomic DNA containing no *Bartonella henselea* DNA; NTC - negative control; reagent (blank control) , P1, P2 , P3 , P4 - DNA of the tested patient.

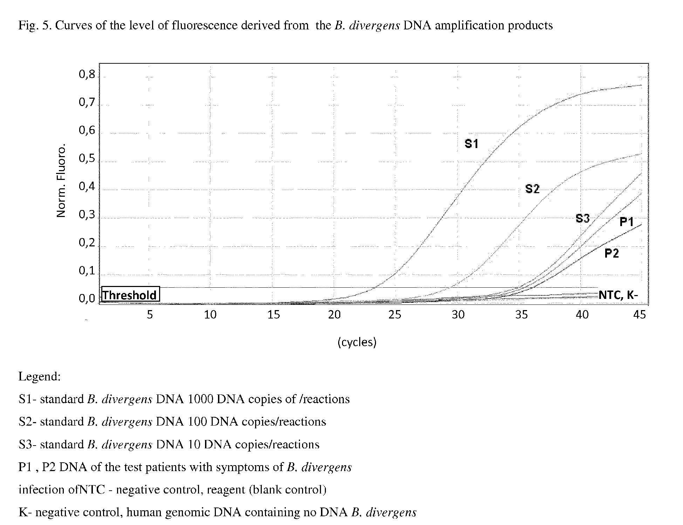

**Figure 5** shows the fluorescence level curves derived from the products of *Babesia divergens* DNA amplification. Abbreviations: S1 - standard *Babesia divergens* DNA 1000 DNA copies /reactions; S2 - standard *Babesia divergens* DNA100 DNA copies/reactions; S3 - standard *Babesia divergens* DNA 10 DNA copies/reactions; K- negative control, human genomic DNA containing no *B.divergens* DNA, NTC - negative control, reagent (blank control); P1 , P2, DNA of the tested patients.

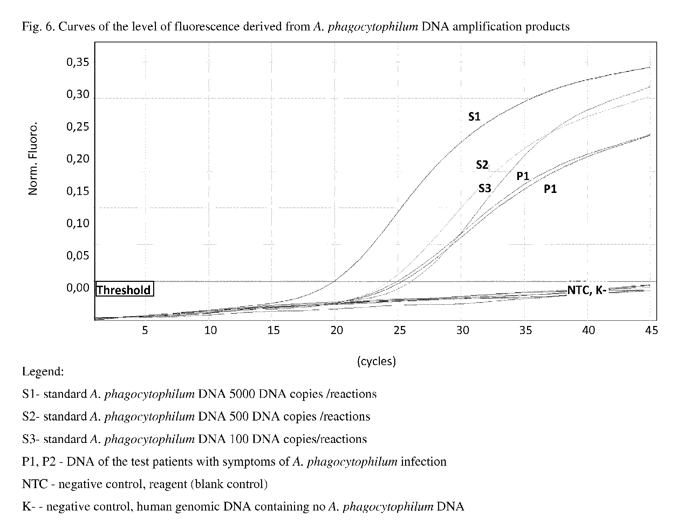

**Figure 6** shows the fluorescence level curves derived from the products of *Anaplasma phagocytophilum* DNA amplification. Abbreviations: S1 - *Anaplasma phagocytophilum* standard DNA 5000 DNA copies/reactions; S2 - standard *Anaplasma phagocytophilum* DNA 500 DNA copies/reactions; S3 - standard *Anaplasma phagocytophilum* DNA 100 DNA copies/reactions; K- negative control, human genomic DNA containing no *A. phagocytophilum* DNA; NTC - negative control, reagent (blank control); P1, P2 , P3 -DNA of tested patients.

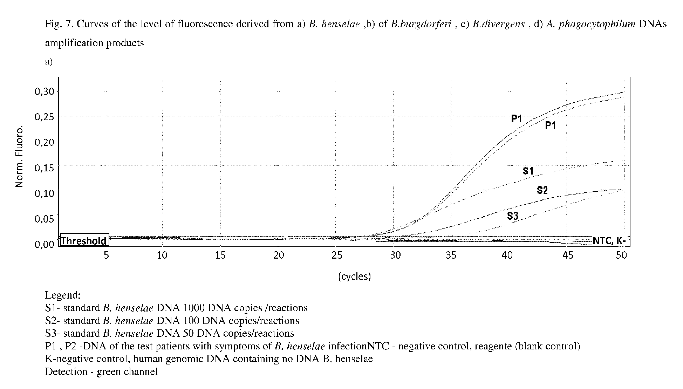

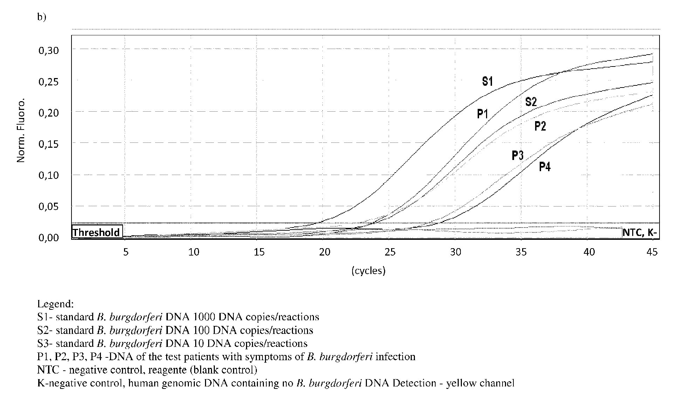

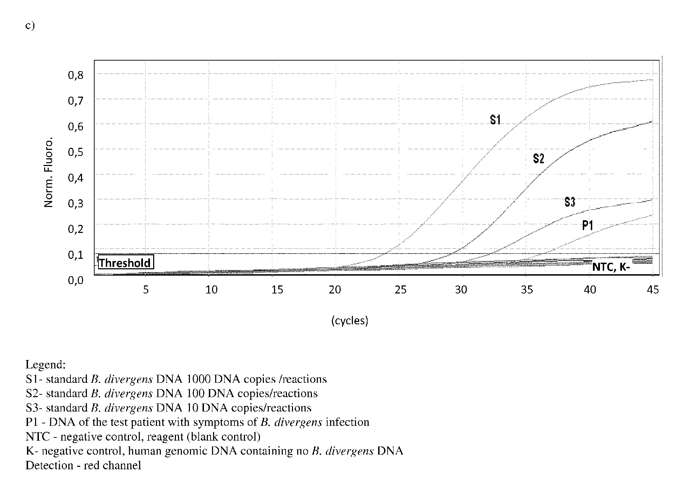

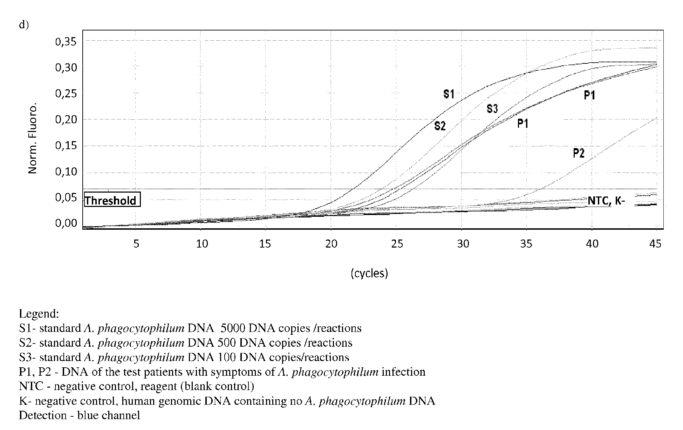

**Figure 7** shows the fluorescence level curves derived from the DNA amplification products a) *B.*
*henselae,* b) of *B.burgdorferi,* c) *B.divergens ,* d) A.phagocytophilum.

### Abbreviations:

S1- standard *B. henselae* DNA 1000 DNA copies/reactions
S2- standard *B. henselae* DNA 100 DNA copies/reactions
S3- standard *B. henselae* DNA 50 DNA copies/reactions
P1, P2 - DNA of the tested patients with symptoms of infection with *B. henselae*
NTC - negative control, reagent (blank control)
K - negative control, human genomic DNA containing no *B. henselae* DNA.
Detection - green channel
S1 - standard *B. burgdorferi* DNA 1000 DNA copies/reactions
S2 - standard *B. burgdorferi* DNA 100 DNA copies/reactions
S3 - standard *B. burgdorferi* DNA 10 DNA copies/reactions
P1, P2, P3 , P4 - DNA of the tested patients with symptoms of infection with *B. burgdorferi*
NTC - negative control, reagent (blank control)
K - negative control, human genomic DNA containing no *B. burgdorferi* DNA
Detection --yellow channel
S1- standard *B. divergens* DNA 1000 DNA copies /reactions
S2- standard *B. divergens* DNA 100 DNA copies/reactions
S3 - standard DNA of *B. divergens* 10 DNA copies/reaction
P1 - DNA of the tested patients with symptoms of infection with *B. divergens*
NTC - negative control, reagent ( blank control)
K - negative control, human genomic DNA containing no DNA *B. divergens*
Detection - red channel
S1- standard *A. phagocytophilum* DNA 5000 DNA copies/reactions
S2 - standard *A. phagocytophilum* DNA 500 DNA copies /reactions
S3 - standard A. *phagocytophilum* DNA 100 DNA copies/reactions
P1, P2 - DNA of the tested patients with symptoms of infection *A. phagocytophilum*
NTC - negative control, reagent ( blank control)
K- negative control, human genomic DNA containing no *A. phagocytophilum* DNA
Detection - blue channel

**The sequences for which the primers and probes were prepared are indicated in the sequence listing**
**SEQ. ID. NO.:43** shows the nucleotide sequence of the recA gene of *B.burgdorferi,* to which the described oligonucleotide primers and probes having sequences listed in Tables 1 or 2 or 3 are complementary.
**SEQ. ID. NO.:44** shows the nucleotide sequence of the ribC gene of *B.henselae* to which the described oligonucleotide primers and probes having sequences listed in Tables 4, 5, or 6 are complementary. **SEQ. ID. NO.:45** shows the nucleotide sequence of the beta-tubulin gene of *B.divergens,* to which the described oligonucleotide primers and probes having sequences listed in Tables 7 or 8 or 9 are complementary.
**SEQ. ID. NO.:46** shows the nucleotide sequence of the gltA gene of *A.phagocuthophilum,* to which the described oligonucleotide primers and probes having sequences listed in Tables 10 or 11 or 12 are complementary.

The following examples are presented only in order to better explain the various aspects of the invention and should not be equated with its whole scope , which is defined in the appended claims.

### Examples

### Example 1. A method of detecting B.burgdorferi DNA in a test sample

This example shows a method of detecting *B.burgdorferi* in a sample containing bacteria or only its DNA (preferably in a peripheral blood sample), which consists of the following steps:

### I. Isolation of the total (human and bacterial) DNA from the test sample

The patient's peripheral blood was analyzed. Genomic DNA of the patient was isolated from 200 µl of peripheral blood with EDTA addition. Isolation of gDNA was performed using the NucleoSpin DxBlood kit ( Marcherey-Nagel, Germany). The obtained genetic material suspended in 100 µl of elution buffer, served as a template for amplification.

### II. Amplification of B.burgdorferi sequence flanked with primers:

### A) Preparation of the reaction mixture:

1. Adding buffers, reagents, nucleotides, and a thermostable DNA polymerase to the test sample in order to prepare the reaction mixture.
2. Adding at least one pair of the specific oligonucleotide primers and one fluorescently labeled probe B) Polymerase Chain Reaction:
   1. Denaturation of double-stranded DNA molecule of the target *B.burgdorferi* sequence,
   2. Hybridization of the primers to their corresponding complementary sequence present in denatured DNA
   3. Extending DNA fragments flanked with primers,
   4. Reading the fluorescence level
   5. Sequentially repeating steps 1-4 to amplify a sufficient number of copies of the tested *B.burgdorferi* DNA fragments.

### Biological material -a detailed description of the different stages

The sample to be tested may be any sample containing fresh mixed or frozen tissue sample, or a biological fluid, most preferably whole peripheral blood taken on EDTA. The sample is treated in such a way as to isolate or concentrate DNA material from the sample.

Fractions isolated from blood can be: a) whole blood, b) plasma or protein cell pellet after centrifugation of the plasma at 350g for 10 min., c) the transition fraction between the plasma and the erythrocytes fraction after their natural sedimentation (about 3h 4°C) or centrifugation at 250g for 30 min., d) cell fractions (mononuclear cell fraction, polymorphonuclear cells fraction, the fraction of nRBC - nucleated Red Blood Cells)

### Example procedure:

### Isolation of DNA

DNA isolation can be carried out using various methods, such as: phenol method, column method (e.g. Qiagen), magnetic separation (e.g. Nuscana ) and using FTA cards (Whattmann Biosciences) also combining some of them. In the illustrated procedure, the isolation requires at least 100-200 µl of fresh peripheral blood taken on EDTA.

Example procedure for isolation of DNA using the QIAamp DNA Mini Kit:
1. Add 20 µl of proteinase K to 200 µl of whole blood and then 200 µl of lysis buffer AL.
2. Mix and incubate at 56°C for 30 min.
3. Add 200 µl of ethanol (96%), vortex , centrifuge (briefly) .
4. Transfer the liquid content (- 600 µl) into the tubes with columns.
5. Centrifuge at 8000 rpm for 2 min.
6. Transfer the columns to the new collective tubes discarding the used ones.
7. Add 500 µl of buffer AW1 to each of the tubes with columns , centrifuge 8000 rpm, 2 min.
8. Transfer the columns to the new collective tubes discarding the used ones.
9. Add 500 µl of buffer AW1 to each of the tubes with columns, centrifuge 14,000 rpm., 4 min.
10. Transfer the columns to the new Eppendorf tubes (1.5 ml) discarding the used ones (the liquid filtrates). Then add 100 µl of heated to temp.70°C AE buffer to each of the tubes with columns, and incubate at room temperature for 5-10 min., . and then centrifuge at 8,000 rpm. for 1 min.

### PCR reaction

### 1 A set of proposed primers and fluorescently labeled probes in the Real -Time PCR reaction:

Flanking primers contain RA_F and RA_R. The sequences of these primers are shown in Table 1 and Table 2. In PCR amplification reaction TaqMan probe labeled with fluorochromes having the sequence shown in Table 3 was used. Using of other pairs of primers , not listed in the table, may be also considered. It should be taken into account that the flanking primers and the probe can be longer or shorter by adding or
deleting one or more nucleotides at both or one end. It should also be taken into account that described primer pairs may be freely combined in one reaction mixture. One of the possible exemplary sets is shown in Table 13.

### 2. Polymerase Chain Reaction

The exemplary composition of the reaction mixture is shown in Table 14. The primer set shown in Table 13 was used.

**Table 14. The exemplary composition of the reaction mixture for PCR**

| **Component** | ***Volume (µl)*** | **The final content of the component in 50 µl of reaction mixture** |
|---|---|---|
| 10x buffer | 5.0 µl | 1x |
| DNTPs (2mM) | 2.0 µl | 3.0 pmol |
| Taq polymerase (5U/µl) | 0.25-0.75 µl | 1.25-4 U |
| Primers RA_F2 and RA_R9 (10 µM each) | 0.75 | 7.5 pmol each |
| Probe RA_PR6 (10 µM) | 0.5 | 5 pmol |
| DNA | 3-5 µl | |
| DdH₂O | | |
| **TOTAL** | **50.0 µl** | |

| | | |
|---|---|---|
| PCR parameters : 95°C for 2 min. Followed by 45 thermal cycles comprising: a ) 95 °C for 30 sec , b) 58-63 °C for 30 sec , c) 72 °C for 35 sec* * - reading of fluorescence level on the yellow channel | | |

3. Reactions were carried out in an apparatus for Real -Time PCR (e.g. RotorGene 6000, the company Corbet Research, Australia). Reaction course and fluorescence emission level derived from the amplified fragments of B.burgdorferi recA gene from patients with erythema migrans and patients with articular symptoms are illustrated in Fig. 1.

### Example 2

### A method of detecting B.burgdorferi DNA in a test sample without DNA isolation step

This example illustrates a method for detecting *B.burgdorferi* in a sample containing bacteria, or only its DNA ( preferably in a sample of peripheral blood or other body fluid sample), comprising the following stages. In the example in the amplification of *B.burgdorferi* sequences flanked with primers was used. Material for the study:

### 1. Dried blood drop on the transport substrate

In case of using transport cards with a blood drop e.g. Whatman 903 Card , 2: Clear GeneSaver Card , 3:FTA Elute Card hydration of dried blood is carried out as a preliminary step prior to PCR. For this purpose, card fragment with an area of 1mm² is cut out and then placed in a test tube with addition of 50 µl of ultra pure water and incubated for 5-10 min. at 50°C. Then the amount of 2-5 µl of extract is used in the PCR reaction.
2. Body fluids - such as whole blood (taken on EDTA, citrate or heparin anticoagulant), cerebrospinal fluid, synovial fluid , and others. If the patient body fluids is used in the PCR reaction, after thorough mixing, it is directly added to the reaction.

Reactions were performed using modified enzymes - DNA polymerases resistant to inhibitor existing in the blood. These enzymes are available in many commercial kits for. PCR e.g. Phusion® Blood Direct PCR Kit from Finnzymes , no. Cat. F- 547S, FailSafe PCR System from EPICENTRA no. Cat. FS99060, OmniTaq and Omni KlenTaq PCR Kit from DNA Polymerase Technology Inc, St. Louis, USA.

A set of proposed primers and fluorescently labeled probes in the Real -Time PCR reaction:
Flanking primers contain RA_F and RA_R. The sequences of these mentioned primers are shown in Table 1 and Table 2, preferably they are labeled with a fluorescent dye at the 5'- end and/or 3'-end or in the middle part of the primer. Examples of primer pairs with corresponding lengths of the amplification products are shown in Table 13. Other, not specified in the table, primer pairs can also be considered. It should be taken into account that the flanking primers can be longer or shorter by adding or deleting one or more nucleotides at both or one end or insertion of the one modified nucleotide or several nucleotides in the middle of the primer sequence. It should also be taken into account that the described pairs of primers may be freely mixed in one reaction mixture. One of many possible examples of primer sets is shown in Table 13.

**Table 15. An example of composition of the reaction mixture to Real -Time PCR using Phusion® Blood Direct PCR Kit from Finnzymes**

| **Component** | ***Volume (µl)*** | **Final content of the component in 50 µl reaction mixture** |
|---|---|---|
| 2x Phusion® Blond PCR Buffer | 25 | 1x |
| Primer RA_F1 | | 0.5µM |
| Primer RA_R1 labeled at the 5 'end with FAM dye | | 0.5µM |
| Phusion® Blond DNA Polymerase | 1 | |
| Whole blood or extract from, transport cards | 2-4 | |
| 50 mM MgCl₂ | 1.5 | |
| 50 mM EDTA | 1.5 | |
| DMSO | 2.5 | 5% |
| DdH₂O | up to 50 µl | |
| **TOTAL** | **50,0 µl** | |

| | | |
|---|---|---|
| PCR parameters: 98°C for 5 min. Followed by 50 cycles comprising a) 98°C for 1 sec, b) about 58-63°C for 5 sec.; c) 72 ° C for 15 sec*. * - reading fluorescence level in the green channel | | |

3. Reactions were carried out in an apparatus for Real -Time PCR ( e.g. RotorGene 6000, the company CorbetResearch, Australia). Reaction course and fluorescence emission level derived from the amplified fragments of *B.burgdorferi* recA gene are illustrated in Fig. 2 .

### Example 3. Detecting B.burgdorferi DNA by Real-Time PCR using intercalating dyes (eg. EvaGreen, SybrGreen, SybrGold) and melting curve analysis of the reaction products

### I. Isolation of B.burgdorferi DNA from a fragment of tissue (biopsy or tick tissue):

Biopsy material of the lesion formed after the tick bite was used for the isolation of bacterial DNA. Isolation of DNA by phenol method from tissues fixed in paraffin blocks and/or ionic liquids and/or biopsy tissue and/or tick tissue.
1. Tissue sections were placed in Eppendorf tubes (1.5 ml).
2. Added to the sections :
   - 300 µl of 2 % SDS in extraction buffer
   - 12 µl of 1 M DTT in the extraction buffer
   - 15 µl proteinase K ( 20 mg/ml )
   The samples were mixed by vortexing and incubated overnight at 56°C on a shaker at 200 rpm.
3. 300 µl of mixture of PCI (phenol/chloroform/isoamyl alcohol 25:24:1 pH 8) was added, vortexed to obtain a milky suspension (at least 15 seconds) and then centrifuged for 5 min at 14,000 rpm.
4. The upper aqueous extract phase was collected (without disturbing the interphase and the bottom organic phase) and placed in the new Eppendorf tubes (1.5 ml).
5. 700 µl of 96% (-20°C) alcohol were added to the tubes and gently mixed by repeated inversion.
6. The samples were then centrifuged for 5 min. at 14,000 rpm.
7. The supernatant was decanted, and washed with 70% ethanol (-20°C), the pellet was briefly centrifuged, alcohol was pipetted and incubated at 37°C until it completely evaporated.
8. The pellet was resuspended in 100 µl or 50 µl of Tris buffer.

### II. Preparation of the reaction mixture for Real-Time PCR

**Table 16. An example of the composition of the reaction mixture for Real-Time PCR**

| **Component** | ***Volume (µl)*** | **The final content of the component in 50 µl reaction mixture** |
|---|---|---|
| 10x buffer | 5.0 µl | 1x |
| dNTPs (2mM) | 2.0 µl | 3.0 pmol |
| Taq polymerase (5U/µl) | 0.25-0.75 µl | 1.25-4 U |
| Primers RA_F10 i RA_R8 (10 µM each) | 0.75 | 7.5 pmol each |
| EvaGreen (2 µM) | 1 | 2 pmol |
| DNA | 3-5 µl | |
| DdH₂O | up to 50 | |
| **TOTAL** | **50.0 µl** | |

| | | |
|---|---|---|
| PCR parameters : 95°C for 2 min, followed by 50 thermal cycles comprising: a) 95°C for 30 sec , b) 58-63°C for 30 sec, c) 72°C for 35 sec | | |

After completed reaction PCR products are subjected to gradual thermal denaturation during which the fluorescence level is read in a specific channel dependent on the used dye (for SybrGreen, SybrGold and EvaGreen - reading of fluorescence level in the green channel or HRM channel) in the camera for Real-Time PCR. As a result of analysis a graph for melting curves of PCR products is generated indicating a presence the specific PCR product characterizing for a tested fragment of the sequence of recA *B.burgdorferi* Fig. 3

### Example 4. A method for detecting B.henselae DNA in a test sample

This example illustrates a method for detecting *B.henselae* in a sample containing bacterium or only its DNA ( preferably, in a sample of peripheral blood), comprising the following steps:
- Isolation of total ( human and bacterial) DNA from the test sample;

The patient's peripheral blood was analyzed. Genomic DNA of the patient was isolated from 200 µl of peripheral blood with EDTA. Isolation of gDNA was performed using NucleoSpin Dx Blood kit (Marcherey -Nagel, Germany). The obtained genetic material suspended in 100 µl of elution buffer served as a template for amplification. - Amplification of B.henselae sequence flanked with primers analogously to Example 1:

### Example procedure:

### Isolation of DNA

DNA isolation can be carried out using various methods, such as: phenol method, column method (e.g. Qiagen), magnetic separation (eg. Nuscana), and using FTA cards (Whattmann Biosciences ) also combining some of them. In the presented procedure for the isolation at least 100-200 µl of fresh peripheral blood collected on EDTA is required.

Example procedure for isolation of DNA using the QIAamp DNA Mini Kit:
1. Add 20 µl of proteinase K and then 200 µl of lysis buffer AL to 200 µl whole blood.
2. Mix and incubate at 56 °C for 30 min.
3. Add 200 µl of ethanol ( 96%), vortex , centrifuge (briefly) .
4. Transfer the liquid content (∼ 600 µl) into the tubes with columns.
5. Centrifuge at 8000 rpm for 2 min.
6. Transfer the columns to the new collective tubes discarding the used ones
7. Add 500 µl of buffer AW1 to each of the tubes with columns , centrifuge 8000 rpm, 2 min.
8. Transfer the columns to the new collective tubes discarding the used ones.
9. Add 500 µl of buffer AW2 to each of the tubes with columns, centrifuge with 14,000 rpm, 4 min. Transfer the columns to the new Eppendorf tubes (1.5 ml) discarding the used tubes (the liquid filtrates).Then add 100 ml of buffer AE heated to a temperature of 70°C to each of the tubes with columns, incubating at room temperature for 5-10 min, and then centrifuge at 8,000 rpm for 1 min.

### PCR reaction

### 1. A set of proposed primers and fluorescently labeled probes in the reaction Real -Time PCR:

Flanking primers contain BH_F1 and BH_R1. The sequences of these mentioned primers are shown in Table 4 and Table 5. In the PCR reaction TaqMan probes labeled with fluorochromes having of the sequence shown in Table 6 were used. It should be taken into account that the flanking primers and probe can be longer or shorter by adding or deleting one or more nucleotides at both or one end.

It should also be taken into account that the described pair of primers can be freely mixed with other pairs of primers in one reaction mixture.

### 2. Polymerase Chain Reaction

An example of the reaction mixture composition is shown in Table 17.

**Table 17. An exemplary composition of the reaction mixture for PCR**

| **Component** | ***Volume (µl)*** | **The final content of the component in 50 µl reaction mixture** |
|---|---|---|
| 10 x buffer | 2.5 µl | 1x |
| DNTPs (5mM) | 1.2 µl | 3.0 pmol |
| MgCl₂ | 1.5 | |
| Taq polymerase (5U/µl) | 0.2 µl | 1 U |
| Primers BH_F1 i BH_R1 (10 µM each) | 0.75 | 7.5 pmol each |
| Probe BH_Pr1 (10 µM) | 0.17 | 2 pmol |
| DNA | 3-5 µl | |
| DdH₂O | up to 25 µl | |
| **TOTAL** | **25.0 µl** | |

| | | |
|---|---|---|
| PCR parameters : 95°C for 2 min. Followed by 45 thermal cycles comprising: a ) 95°C for 20 sec , b) 58°C for 20 sec, c) 72°C for 20 sec * 10 * - fluorescence level reading in the green channel | | |

The reactions take place in the Real- Time PCR apparatus (eg. RotorGene 6000, the company CorbetResearch , Australia).

Reactions course and fluorescence emission level derived from the amplified *B.henselae rib*C gene fragments are illustrated in Fig. 4

### Example 5. A method of detecting B.divergens DNA in a test sample

The example presents a method of detecting *B.divergens* in a sample containing bacteria or only the its DNA (preferably in a peripheral blood sample), comprising the following steps:
- Isolation of total ( human and bacterial ) DNA from the sample
   The patient's peripheral blood were analyzed. Genomic DNA of the patient were isolated from 200 µl of peripheral blood with EDTA. Isolation of gDNA was performed using the NucleoSpin kit Dx Blood 20 ( Marcherey-Nagel, Germany). The resulting genetic material suspended in 100 µl of elution buffer, served as a template for amplification.
- Amplification *B.divergens* sequences flanked with primers analogously to Example 1:

### Example procedure:

### Isolation of DNA

DNA isolation can be carried out using various methods, such as: phenol methods, column method (e.g., Qiagen), magnetic separation (eg. Nuscana), and using FTA cards (Whattmann Biosciences) also combining some of them. The presented procedure for the isolation requires at least 100-200 µl of fresh peripheral blood collected on EDTA.

Example procedure for isolation of DNA using the QIAamp DNA Mini Kit:
1 Add 20 µl of proteinase K and then 200 µl of lysis buffer AL to 200 µl whole blood.
2. Mix and incubate at 56°C for 30 min.
3. Add 200 µl of ethanol (96%), vortex , centrifuge (briefly).
4. Transfer the liquid content (∼ 600 µl) to the tubes with columns.
5. Centrifuge at 8000 rpm for 2 min.
6. Transfer the columns to the new collective tubes discarding the used ones.7. Add of 500 µl of buffer AW1 to each of the tubes with columns, centrifuge 8000 rpm, 2 min.
8. Transfer the column to a new collective tubes discarding the used ones .
9. Add 500 µl of AW2 buffer to each of the tubes with columns , centrifuge 14,000 rpm, 4 min.
10. Transfer the colums to the new Eppendorf tubes (1.5 ml) discarding the used ones ( with liquid filtrates ). Then add 100 µl buffer heated to temp.70°C to each of the tubes with columns , incubate at room temperature for 5-10 min., and then centrifuge at 8000 rpm for 1 min.

### PCR reaction

### 1. A set of proposed primers and fluorescently labeled probes in the reaction Real -Time PCR:

Flanking primers contain Bd_F1 and Bd_R1. The sequences of these primers are shown in Table 7 and Table 8. TaqMan probes labeled with fluorochromes having the sequence shown in Table 9 was used in the PCT reaction. It should be taken into account that the flanking primers and probe may be longer or shorter by adding or deleting one or more nucleotides at both or one end.

It should also be taken into account that the described pair of primers can be freely mixed with other pairs of the primers in the one reaction mixture

### 2.Polymerase Chain Reaction

Example of the reaction mixture composition is shown in Table 18.

**Table 18. An exemplary composition of the reaction mixture for PCR**

| **Component** | ***Volume (µl)*** | **The final content of the component in 50 µl of reaction mixture** |
|---|---|---|
| 10x buffer | 2.5 µl | 1x |
| DNTPs (5mM) | 1.5 µl | 3.0 pmol |
| MgCl2 | 1.2 | |
| Taq polymerase (5U/µl) | 0.2 µl | 1 U |
| Primers Bd_F1 i Bd_R1(10 µM each) | 0.75 | 7.5 pmol each |
| Probe Bd_PR1 (10 µM) | 0.17 | 2 pmol |
| DNA | 3-10 µl | |
| DdH₂O | up to 25µl | |
| **TOTAL** | **25.0 µl** | |

| | | |
|---|---|---|
| PCR parameters: 95°C for 2 min. Followed by 45 thermal cycles comprising: a ) 95°C for 15 sec , b) 57°C for 20 sec , c) 72°C for 20 sec * * - fluorescence level reading in the red channel | | |

3. Reactions were carried out in the apparatus for Real -Time PCR ( e.g. RotorGene 6000, the company CorbetResearch, Australia). The reaction course and the fluorescence emission level derived from the amplified fragments of *B. divergens* gene are illustrated in Fig. 5

### Example. 6 A method for detecting Anaplasma phagocytophilum DNA in the test sample.

The example presents a method for detecting *A.phagocytophilum* in a sample containing bacteria or only its DNA ( preferably in a peripheral blood sample), comprising the following steps:
Isolation of total (human and bacterial ) DNA from the sample. The patient's peripheral blood was analyzed. Genomic DNA of the patient was isolated from 200 µl of peripheral blood with EDTA.
Isolation of gDNA was performed using the NucleoSpin kit Blo15 Dx from Marcherey-Nagel, Germany.

The resulting genetic material suspended in 100 µl of elution buffer, served as a template for amplification.

Amplification *A.phagocytophilum* sequences flanked with primers analogously to Example 1:

### Example procedure:

### Isolation of DNA

DNA isolation may be performed using different methods, such as: phenol method, column method (e.g., Qiagen), magnetic separation (e.g. Nuscana), and using FTA cards ( Whattmann Biosciences ) also combining some of them. The presented procedure for the isolation requires at least 100-200 µl of fresh peripheral blood collected on EDTA.

Example procedure for isolation of DNA using the QIAamp DNA Mini Kit:
1. Add 20 µl of proteinase K and then 200 µl lysis buffer AL to 200 µl whole blood.
2. Mix and incubate at 56°C for 30 min.
3. Add 200 µl of ethanol ( 96%), vortex , centrifuge (briefly).
4. Add liquid content (∼ 600 µl) to the tubes with columns.
5. Centrifuge at 8000 rpm for 2 min.
6. Transfer columns to the new collective tubes discarding the used ones.
7. Add 500 µl of AW1 buffer to each of the tubes with columns , centrifuge 8,000 rpm, 2 min.
8. Transfer columns to the new collective discarding the used ones.
9. Add 500 µl of buffer AW2 to each of the tubes with columns , centrifuge 14,000 rpm, 4 min.
10. Transfer columns to the new Eppendorf tubes (1.5 ml) discarding the used ones (with liquid filtrates). Then add 100 µl buffer heated to temp. about 70° C to each of the tubes with columns , incubate at room temperature for 5-10 min, and then centrifuged at 8,000 rpm for 1 min.

### PCR reaction

### 1. A set of proposed primers and fluorescently labeled probes in the reaction Real -Time PCR:

Flanking primers contain Bd_R1 and Bd_F1 contain. The sequences of these primers are shown in Table 7 and Table 8. TaqMan probes labeled with fluorochromes having the sequence shown in Table 9 was used in the PCT reaction. It should be taken into account that the flanking primers and the probe may be longer or shorter because of adding or deleting one or more nucleotides at both or one end.

It should also be taken into account that described pair of primers can be freely mixed with other pairs of the primers in one reaction mixture.

### 2. Polymerase Chain Reaction

The exemplary composition of the reaction mixture is shown in Table 19:

**Table 19. Exemplary composition of the reaction mixture for PCR**

| **Component** | ***Volume (µl)*** | **The final content of the component in 50 µl reaction mixture** |
|---|---|---|
| 10x buffer | 2.5 µl | 1x |
| DNTPs (5mM) | 1.5 µl | 3.0 pmol |
| MgCl2 | 1.2 | |
| Taq polymerase (5U/µl) | 0.2 µl | 1 U |
| Primers Ap_F1 i Ap_R1(10 µM each) | 0.75 | 7.5 pmol each |
| Probe Ap_Pr1 (10 µM) | 0.17 | 2 pmol |
| DNA | 3-10 µl | |
| DdH₂O | up to 25ul | |
| **TOTAL** | **25.0 µl** | |

| | | |
|---|---|---|
| PCR parameters : 95 for 2 min at 0C. Followed by 45 thermal cycles comprising: a ) 95°C for 15 sec , b) 57°C for 20 sec , c) 72°C for 20 sec * * - fluorescence level reading in the blue channel | | |

The reactions take place in the apparatus for Real- Time PCR (e.g. RotorGene 6000, the company CorbetResearch , Australia).

Reaction course and fluorescence emission level derived from
the amplified *A.phagocytophilum* gene fragments are illustrated in Fig. 6.

### Example 7. The method of simultaneous detection of DNAs B.burgdorferi, B.henselae, B.divergens and Anaplasma phagocytophilum in the test sample

This example shows the method for simultaneous detection of *B.burgdorfer, B.henselae, B.divergens* and *Anaplasma phagocytophilum* in a sample containing dead or alive microorganisms or only their DNA (preferably in a peripheral blood sample) comprising the following steps :
- Isolation of total (human and bacteria ) DNA from the sample
   The patient's peripheral blood was analyzed. Genomic DNA of the patient was isolated from 200 µl of peripheral blood with EDTA. Isolation of gDNA was performed using the kit NucleoSpin Dx Blood 15 (Marcherey-Nagel, Germany). The resulting genetic material suspended in 100 µl of elution buffer served as a template for amplification.
- Amplification sequences flanked with the primers :

### Example procedure:

### - Isolation of DNA

DNA isolation may be performed using different methods , such as: phenol method, column method (e.g. Qiagen), magnetic separation (e.g. Nuscana ), and using FTA cards ( Whattmann Biosciences ) also combining some of them. The presented procedure for the isolation requires at least 100-200 µl of fresh peripheral blood collected on EDTA.

Example procedure for isolation of DNA using the QIAamp DNA Mini Kit:
1. Add 20 µl of proteinase K and then 200 µl of lysis buffer AL to 200 µl whole blood.
2. Mix and incubate at 56 ° C for 30 min.
3. Add 200 µl of ethanol ( 96%), vortex , centrifuge (briefly).
4. Transfer the liquid content (∼ 600 µl) tubes with columns.
5. Centrifuge at 8000 rpm for 2 min.
6. Transfer the columns to the new collective tubes discarding the used ones.
7. Add 500 µl of AW1 buffer to each of the tubes with columns , centrifuge 8,000 rpm, 2 min.
8. Transfer the columns to the new collective tubes discarding the used ones.
9. Add 500 µl of AW2 buffer to each of the tubes with columns , centrifuge 14,000 rpm, 4 min.
10. Transfer the columns to new Eppendorf tubes (1.5 ml) discarding the used ones used (the liquid filtrates). Then add 100 µl buffer heated to at 70°C to each of the the tubes with columns , incubate at room temperature for 5-10 min. and then centrifuge at 8,000 rpm for 1 min.

### PCR reaction

### 1. A set of proposed primers and fluorescently labeled probes in the reaction Real -Time PCR:

Flanking primers comprise a mixture of: BB_F1 to BB_F10, BB_R1 to BB_R16, BH_F1, BH_R1, - Bd_F1, Bd_R 1, Ap_F1 and Ap_R1. The TaqMan probes BB_PR3 , BH_Pr1, Bd_Pr1 and Ap_Pr1 labeled with different fluorochromes allow detection on 4 different channels ( green, yellow , red and blue). It should be taken into account that flanking primers and probes may be made longer or shorter by adding or deleting one or more nucleotides at both or one end.

### 2. Polymerase Chain Reaction

Example of the reaction mixture composition is shown in Table 20

**Table 20. An exemplary composition of the reaction mixture for PCR**

| **Component** | ***Volume (µl)*** | **The final content of the component in 50 µl reaction mixture** |
|---|---|---|
| 10x buffer | 2.5 µl | 1x |
| DNTPs (5mM) | 3 µl | 6.0 pmol |
| MgCl₂ | 1. 8 | |
| Taq polymerase (5U/µl) | 0.6 µl | 3 U |
| Primers BB_F1, BB_R1, BH_F1,BH_R1, Bd_F1, Bd_R1, Ap_F1, Ap_R1 (10 µM each) | 0.75 | 7.5 pmol each |
| Probes BB_PR3, BH_Prl, Bd_Pr1 and Ap_Pr1 (10 µM) | 0.15 | 2 pmol |
| DNA | 3-10 µl | |
| DdH₂O | up to 25µl | |
| **TOTAL** | **25.0 µl** | |

| | | |
|---|---|---|
| PCR parameters : 95°C for 4 min. Followed by 45 thermal cycles comprising: a) 95°C for 15 sec , b) 58°C for 20 sec , c) 72°C for 20 sec* * fluorescence level reading on all the four channels | | |

3. Reactions were carried out in the apparatus for Real -Time PCR ( e.g. RotorGene 6000, the company CorbetResearch, 20 Australia). Reaction course and fluorescence emission levels derived from of the amplified fragments of *B.burgdorferi, B.henselae, Anaplasma phagocytophilum,* and *B.divergens* genes are illustrated in Fig. 7a, b, c, and d.

### Literature

1. Burrascano, Jr., M.D -Diagnostics Hints and Treatment Guidelines for Tick Borne Illnesses by. - 12th Edition, 1998.
2. Burgdorfer, W., 1986, Rev. Infect. Dis., 6: 932-940.
3. Liebling et al., "The Polymerase Chain Reaction for the Detection of Borrelia burgdorferi in Human Body Fluids," Arth Rheum 36(5): 665-675, 1993.
4. Saiki RK i wsp. 1985, Enzymatic amplification of beta-globin genomic sequences and restriction site analysis for diagnosis of sickle cell anemia Science, Dec 20;230(4732):1350-4.
5. Saiki RK, i wsp. 1988, Primer-directed enzymatic amplification of DNA with a thermostable DNA polymerase. Science. 239:487-491.
6. Schmidt et al., "Detenction of Borrelia burgdorferi DNA by Polymerase Chain Reaction in the Urine and Breast Milk of Patients with Lyme Borreliosis," Diagn. Microbiol. Infect. Dis. 21:121-128, 1995.
7. Schwaiger et al., "Routine diagnosis of Borrelia burgdorferi (sensu lato) infections using a real-time PCR assay" Clin. Microbiol. Infect. 7(9): 461-9, 2001.
8. Steere AC. 2001; Lyme disease. N England J Med. 345:115-124.
9. WO2004005479 A high resolution typing systems for pathogenic Borrelia.
10. US Patent Application 20070172829 Oligonucleotides and methods for detecting *Borrelia burgdorferi* bialko FLA.
11. WO9635450 Diagnostic tests for new *Spriochete, Borrelia lonesteri.*
12. US patent Sensitive diagonostic test for Lyme disease.
13. EP 0200362 Process for amplifying, detecting and/or cloning nucleic acid sequences.
14. EP 0201184 Process for amplifying nucleic acid sequences.

## Claims

1. An oligonucleotide comprising at least 15 consecutive nucleotides comprised by the oligonucleotide derived from the recA sequence of *Borrelia burgdorferi* ,selected from a group of oligonucleotides comprising RA_F1 to RA_F10 having sequences shown in SEQ ID NO: 1 to SEQ. ID. NO.: 10 or RA_R1 to RA_R16 having sequences shown in SEQ. ID. NO.:11 to SEQ. ID. NO.:27 or RA_PR1 to RA_PR7 having the sequences presented in SEK ID NO: 28 to SEQ. ID. NO.:33 and the complementary sequences thereof.

2. An oligonucleotide comprisingat least 15 consecutive nucleotides comprised by the oligonucleotides derived from the ribC sequence of *Bartonella henselae,* selected from a group of oligonucleotides comprising BH_-F1 having the sequence shown in SEQ. ID. NO.:34, BH_R1 having the sequence shown in SEQ. ID. NO.:35 and BH_Pr having the sequence shown in SEQ. ID. NO.:36 and the complementary sequences thereof.

3. An oligonucleotide comprising at least 15 consecutive nucleotides comprised by the oligonucleotide derived from beta-tubulin gene sequence of *Babesia divergens,* selected from a group of oligonucleotidescomprising Bd_F1 having the sequence shown in the SEQ. ID. NO.:37, Bd_R1 having the sequence shown in SEQ. ID. NO.:38 and Bd_Pr1 having the sequence shown in SEQ. ID. NO.:39 and the complementary sequences thereof.

4. An oligonucleotide comprising at least 15 consecutive nucleotides comprised by the oligonucleotide derived from the gltA gene sequence of *Anaplasma phagocytophilum,* selected from a group of oligonucleotides comprising Ap_F1 having sequence shown in SEQ. ID. NO.:40, Ap_R1 having the sequence shown in SEQ.ID.NO.::41 and Ap_Pr1 having sequence shown in SEQ. ID. NO.:42 and the complementary sequences thereof.

5. The oligonucleotide of claim 1 to 4, **characterized in that** it is having the sequence of one of the oligonucleotides shown in SEQ. ID. NO.: 1 to SEQ. ID. NO.:42.

6. The oligonucleotide of claim 1 to 4 , **characterized in that** it is labeled, in particular fluorescently labeled with at least one fluorescent label at the 5 '-end and/or 3'-end or in the middle part of the sequence.

7. A method for detecting the presence and quantity of *Borrelia burgdorferi* DNA in the test biological sample by Real -Time PCR, **characterized in that** it comprises:
a) sample preparation;
b) DNA isolation;
c) amplification of a fragment of at least one sequence present in the genome of *Borrelia burgdorferi,* using DNA contained in the test sample as a template, wherein the recA gene-specific primers are oligonucleotide pair RA-F and RA_R selected from oligonucleotides comprising at least 15 contiguous nucleotides from RA_F1 to RA_F10 and RA_R1 to RA_R16 having the sequences of SEQ ID NO: 1 to SEQ.ID.NO.:10 and SEQ. ID. NO.:11 to SEQ. ID. NO.:27;
d) identifying the increase of the specific reaction products by detecting fluorescence level derived from the probes selected from a group of fluorescently labeled oligonucleotides RA_PR1 to RA_PR7 having the sequences shown in SEQ. ID. NO.:28 to SEQ.ID.NO.: 33, and
e) optionally determining the number of DNA copies of the test microorganism in a biological sample by comparing with a standard curve.

8. A method for detecting the presence of *Borrelia burgdorferi* DNA by Real-Time PCR , **characterized in that** it comprises:
a) sample preparation;
b) DNA isolation;
c) amplification of a fragment of at least one sequence present in the genome of *Borrelia burgdorferi,* using DNA contained in the test sample as a template, wherein the recA gene-specific primers are the oligonucleotide pair RA_F and RA_R, wherein one is selected from oligonucleotides comprising at least 15 consecutive nucleotides of RA_F1 to RA_F10 having the sequences of SEQ ID NO: 1 to SEQ. ID. NR: 10 and the second one is selected from a group consisting of RA_R1 to RA_R16 having the sequences presented in SEQ. ID. NO.:11 to SEQ. ID. NO.:27;
d) identification of an increase of the specific reaction products by detecting fluorescence level derived from the intercalating fluorescent dyes, located in the the 5'-end and/or 3'-end or in the middle of the sequence of oligonucleotides used for the PCR reaction i.e. oligonucleotides having the sequences of SEQ ID NO: 1 to SEQ.ID.NO.:10 and SEQ. ID. NO.:11 to SEQ. ID. NO.:27, and
e) optionally determining the number of DNA copies of the test microorganism in a biological sample by comparing with a standard curve.

9. A method for detecting the presence and quantity of *B.henselae* DNA in a test biological sample by Real -Time PCR technique , **characterized in that** it comprises:
a) sample preparation;
b) DNA isolation;
c) amplification of a fragment of at least one sequence present in the genome of *B.henselae,* using DNA contained in the sample as a template, wherein ribC gene-specific the primers are the oligonucleotide pair BH_F1 having the sequence shown in the SEQ. ID. NO.:34 and BH_R1 having the sequence shown in SEQ. ID. NO: 35, comprising at least 15 contiguous nucleotides from these sequences;
d) identification of an increase of the specific reaction products by detecting fluorescence level derived from BH_Pr probe having the sequence illustrated in SEQ. ID. NO.:36, and
e) optionally determining the number of a DNA copies of the test microorganism in a biological sample by comparing with a standard curve.

10. A method for detecting the presence of *B.henselae* DNA by Real-Time PCR technique, **characterized in that** it comprises:
a) sample preparation;
b) DNA isolation;
c) amplification of a fragment of at least one sequence present in the genome of *B.henselae,* using DNA contained in the sample as a template, wherein the ribC gene-specific primers are oligonucleotide pair BH_F1 having the sequence shown in the SEQ. ID. NO.:34 and BH_R1 having of the sequence shown in SEQ. ID. NO.:35, comprising at least 15 contiguous nucleotides from these sequences;
d) identification of an increase of specific reaction products by detecting fluorescence level derived from the intercalating fluorescent dyes, located at the 5'-end and/or 3'-end or in the middle of the of oligonucleotide sequence used for the PCR reaction i.e. the oligonucleotides having the sequences of SEQ. ID. NO.:34 and SEQ. ID. NO.: 35, and
e) optionally determining the number of DNA copies of the test microorganism in a biological sample by comparing with a standard curve.

11. A method for detecting the presence and quantity of *Babesia divergens* DNA by Real-Time PCR technique in a test biological sample, **characterized in that** it comprises:
a) sample preparation;
b) DNA isolation;
c) amplification of a fragment of at least one sequence present in the genome of B divergens, using DNA contained in the sample as a template, wherein beta-tubulin gene-specific primers are the oligonucleotide pair Bd_F1 having the sequence shown in SEQ. ID. NO.:37 and Bd_R1 having the sequence shown in SEQ. ID. NR: 38, comprising at least 15 consecutive nucleotides of the sequences;
d) identification of an increase of the specific reaction products by detecting fluorescence level derived from the Bd_Pr probe having the sequence illustrated in SEQ. ID. NO.:39, and
e) optionally determining the number of DNA copies of the test microorganism in a biological sample by comparing with a standard curve.

12. A method for detecting the presence of *Babesia divergens* DNA by Real - Time PCR technique, **characterized in that** it comprises:
a) sample preparation;
b) DNA isolation;
c) amplification of a fragment of at least one sequence present in the genome of *Babesia divergens,* using DNA contained in the test sample as a template, wherein the beta-tubulin gene-specific primers are oligonucleotide pair Bd_F1 having the sequence shown in SEQ. ID. NO.:37 and Bd_R1 having the sequence shown in SEQ. ID. NO: 38, comprising at least 15 consecutive nucleotides of these sequences;
d) identification of an increase of specific reaction products by detecting fluorescence level derived from the fluorescent intercalating dyes, located at the 5 '-end and/or 3'-end or in the middle of the oligonucleotide sequences used for the PCR reaction i.e. the oligonucleotides having the sequences of SEQ. ID. NO.:37 and SEQ ID NO: 38, and
e) optionally determining the number of DNA copies of the test microorganism in a biological sample by comparing with a standard curve .

13. A method for detecting the presence and quantity of *Anaplasma phagocytophilum* DNA Real -Time PCR technique in a biological test sample , **characterized in that** it comprises:
a) sample preparation;
b) DNA isolation;
c) amplification of a fragment of at least one sequence present in the genome of *Anaplasma phagocytophilum,* using DNA contained in the sample as a template, wherein the gltA gene-specific primers are the oligonucleotide pair Ap_F1 having the sequence shown in SEQ. ID. NO.:40 and Ap_R1 having the sequence shown in SEQ. ID. NO: 41, comprising at least 15 consecutive nucleotides of these sequences;
d) identification of an increase of the specific reaction products by detecting fluorescence level derived from Ap_Pr probe havingthe sequence illustrated in SEQ. ID. NO.:41, and
e) optionally determining the number of DNA copies of the test microorganism in a biological sample by comparing with a standard curve.

14. A method for detecting the presence of *Anaplasma phagocytophilum* DNA Real -Time PCR technique, **characterized in that** it comprises:
a) sample preparation;
b) DNA isolation;
c) amplification of a fragment of at least one sequence present in the genome of *Anaplasma phagocytophilum,* using DNA contained in the sample as a template, wherein the gltA gene-specific primers are the oligonucleotide pair Ap_F1 having the sequence shown in SEQ. ID. NO.:40, Ap_R1 having the sequence shown in SEQ. ID. NO.:41, comprising at least 15 consecutive nucleotides of these sequences;
d) identification of an increase of the specific reaction products by detecting fluorescence level derived from the intercalating fluorescent dyes, located at the 5'-end and/or 3'-end or in the middle of the sequence of oligonucleotides used for the PCR reaction having the sequences of SEQ. ID. NO.:40 and SEQ ID NO: 41, and
e) optionally determining the number of the DNA copies of the test microorganism in a biological sample in comparison with a standard curve.

15. A method for simultaneously detecting the presence of DNAs of *B.burgdorferi* and/or *B.henselae* and/or *B.divergens* and/or *A*. *phagocytophilum* in the biological samples, **characterized in that** it comprises:
a ) total DNA isolation;
b) amplification of the fragments of recA gene sequence , ribC gene sequence, beta-tubulin gene sequence and gltA gene sequence flanked with a mixture of primers RA_F1 to RA_F10, RA_R1 to RA_-R16, - BH_F1, BH_R1, -Bd_F1, Bd_R1 and Ap_F1 and Ap_R1 using fluorescently labeled TaqMan probes RA_PR1 to RA_PR7, BH_Pr1, Bd_Pr1 and Ap_Pr1 in a polymerase chain reaction, wherein the primers and the probes comprise at least 15 consecutive nucleotides of these sequences;
c) the reading fluorescence level and an indication of the number of DNA copies of the test microorganism in the biological sample.

16. Method according to one of the claims 7 to 15, **characterized in that** the used primers are Fluorescently labeled primers.

17. The method according to one of the claims 7 to 15, **characterized in that** the used fluorescent dye is one of the DNA intercalating dyes (SybrGreen, Eva Greek, SybrGold, Syto9) or other one having similar physico-chemical properties.

18. The method according to one of the claims 7 to 15, **characterized in that** the conditions for the amplification comprise initial denaturation at 95°C 1-5 min. then 25-55 thermal cycles with a profile: 95°C -20 s, 58 - 63°C - 20s and 72°C -30 s, and the reading the fluorescence level following each reaction cycle.

19. Method according to one of the claims 7 to 15, **characterized in that** the amplification products are identified by analyzing the fluorescence level derived from a specific PCR product.

20. Method according to one of the claims 7 to 15, **characterized in that** the test sample is derived from the patient's blood, preferably whole blood, serum, plasma or cell-protein precipitate after centrifugation of plasma, buffy coat or cell fraction of blood nucleated cells or cerebrospinal fluid or synovial fluid, or urine, or tissue biopsy.

21. Method according to one of the claims 7 to 15, **characterized in that** the test sample is obtained from tick tissue or animal tissue.

22. Method according to one of the claims 7 to 15, **characterized in that** the test sample is derived from the subject's blood during the course of antibiotic therapy in order to verify the effectiveness of medical treatment.

23. A kit for detecting the presence of DNAs of *B.burgdorferii* and/or *B.henselae,* and/or *B.divergens ,* and/or *A.phagocytophilum,* **characterized in that** it consists of:
at least two different oligonucleotides, each comprising at least 15 consecutivenucleotides, comprised by the oligonucleotide derived from the recA sequence of the genome of the *B. burgdoferi,* one selected from the group consisting of RA_F1 to RA_F10 having the sequences shown in SEQ ID NO: 1 to SEQ. ID. NO.: 10 and the second one selected from RA_R1 to RA_R16 having the sequences shown in SEQ. ID. NO.: 11 to SEQ. ID. NO.:27 and a labeled oligonucleotide selected from RA_PR1 to RA_PR7 having the sequences shown in SEQ. ID. NO.:28 to SEQ. ID. NO.:33, and the complementary sequences thereof and/or at least two different oligonucleotides, each comprising at least 15 consecutive nucleotides, comprised by the oligonucleotide derived from the ribC sequence of the *B.henselae* genome selected from BH_F1 oligonucleotide having the sequence shown in SEQ. ID. NO.:34 and the BH_R1 oligonucleotide having the sequence shown in SEQ. ID. NO.:35 and the labeled BH_Pr having the sequence shown in SEQ. ID. NO.:36 and the complementary sequences thereof and/or at least two different oligonucleotides, each comprising at least 15 consecutive different nucleotides, comprised by the oligonucleotide derived from the beta tubulin gene sequence of the *B.divergens* genome selected from Bd_F1 oligonucleotide having the sequence shown in SEQ. ID. NO.: 37, and Bd_R1 oligonucleotide having the sequence shown in SEQ. ID. NO.:38 and a fluorescently labeled Bd_Pr having the sequence shown in SEQ. ID. NO.:39 and the complementary sequences thereof. and/or at least two different oligonucleotides, each comprising at least 15 consecutive nucleotides, comprised by the oligonucleotide derived from the gltA gene sequence of the of the *A.phagocytophilum* genome selected from Ap_F1 oligonucleotide, having the sequence shown in SEQ. ID. NO: 40, and Ap_R1 oligonucleotide having the sequence shown in SEQ. ID. NO.:41 and the labeled Ap_Pr1 having the sequence shown in SEQ. ID. NO.:42 and the complementary sequences thereof, and further comprises dyes, and optionally also suitable DNA amplification reagents.

24. The kit according to claim 23 for detecting the presence of DNAs of *B.burgdorferi* and/or *B.henselae* and/or *B.divergens* and/or *A.phagocytophilum* in the biological samples of patients with late Lyme boreliosis.

25. A kit according to claim 23 for detecting the presence of DNAs of *B.burgdorferii* and/or *B.henselae* and/or *B.divergens* and/or *A.phagocytophilum* in the biological samples of the patients during the antibiotic therapy in order to verify the effectiveness of therapy.

26. The kit of claim 23 to 25, **characterized in that** the probe or primers are fluorescently labeled.

27. The use of oligonucleotides comprising at least 15 contiguous nucleotides comprised by the oligonucleotide derived from the recA sequence of the *B.burgdorferi* genome, selected from the group consisting of oligonucleotides : RA_F1 to RA_F10 having the sequences shown in SEQ ID NO: 1 to SEQ. ID. NO.: 10 or RA_R1 to RA_R16 having the sequences shown in SEQ. ID. NO.:11 to SEQ. ID. NO.:27 or RA_PR1 to RA_PR7 having the sequences shown in SEQ. ID. NO.:28 to SEQ. ID. NO.:33 and the complementary sequences thereof;
and/or
derived from the ribC sequence of the *B.henselae* genome ,selected from oligonucleotides BH_ F1 having the sequence shown in SEQ. ID. NO.:34, BH_R1 having the sequence shown in SEQ ID NO: 35 and BHPr having the sequence shown in SEQ. ID. NO.:36 and the complementary sequences thereof and/or derived from the beta-tubulin gene sequences from the *B. divergens* genome selected from the Bd_F1 oligonucleotides having the sequence shown in SEQ. ID. NO.:37, Bd_R1 having the sequence shown in SEQ. ID. NO.:38 and Bd_Pr having the sequence shown in SEQ. ID. NO.:39 and the complementary sequences thereof,
and/or
derived from the gltA gene sequence of the *A.phagocytophilum* genome selected from the group consisting of Ap_F1 oligonucleotides having the sequence shown in SEQ. ID. NO.:40, Ap_R1 having the sequence shown in SEQ. ID. NO.:41 and Ap_Pr1 having the sequence shown in SEQ. ID. NO.:42 and the complementary sequences thereof,
for the manufacture of a kit for detecting the presence of DNAs of *B.burgdorferi* and/or *B.henselae* and/or *B.divergens* and/or *A.phagocytophilum* and for diagnosis infections with these microorganism.
